(19) 

Europäisches
Patentamt
European
Patent Office
Office européen
des brevets

(11)  **EP 1 639 003 B1**

(12)  **EUROPEAN PATENT SPECIFICATION**

(45) Date of publication and mention
of the grant of the patent:
**07.04.2010 Bulletin 2010/14**

(51) Int Cl.:
*C07K 14/54* (2006.01)  *C12N 5/074* (2010.01)

(21) Application number: **04737879.9**

(86) International application number:
**PCT/CA2004/000940**

(22) Date of filing: **25.06.2004**

(87) International publication number:
**WO 2004/113380 (29.12.2004 Gazette 2004/53)**

(54) **USE OF CARDIOTROPHIN TO MODULATE STEM CELL PROLIFERATION**

VERWENDUNG VON CARDIOTROPHIN ZUR MODULATION DER
STAMMZELLENPROLIFERATION

UTILISATION DE LA CARDIOTROPHINE POUR MODULER LA PROLIFERATION DES CELLULES
SOUCHES

(84) Designated Contracting States:
**AT BE BG CH CY CZ DE DK EE ES FI FR GB GR
HU IE IT LI LU MC NL PL PT RO SE SI SK TR**

(30) Priority: **25.06.2003 US 482001 P**

(43) Date of publication of application:
**29.03.2006 Bulletin 2006/13**

(73) Proprietor: **Ottawa Hospital Research Institute
Ottawa, ON K1Y 4E9 (CA)**

(72) Inventor: **MEGENEY, Lynn
Ottawa, Ontario K1H 6H9 (CA)**

(74) Representative: **Mallalieu, Catherine Louise et al
D Young & Co
120 Holborn
London EC1N 2DY (GB)**

(56) References cited:
**EP-A- 1 437 365     US-A1- 2004 006 018**

- PENNICA DIANE ET AL: "Cardiotrophin-1:
Biological activities and binding to the leukemia
inhibitory factor receptor/gp130 signaling
complex" JOURNAL OF BIOLOGICAL
CHEMISTRY, vol. 270, no. 18, 1995, pages
10915-10922, XP002297187 ISSN: 0021-9258

- WOLLERT K C ET AL: "CARDIOTROPHIN-1 AND
THE ROLE OF GP130-DEPENDENT SIGNALING
PATHWAYS IN CARDIAC GROWTH AND
DEVELOPEMENT" JOURNAL OF MOLECULAR
MEDICINE, SPRINGER VERLAG, DE, vol. 75, no.
7, 1997, pages 492-501, XP001008240 ISSN:
0946-2716

- PENNICA D ET AL: "EXPRESSION CLONING OF
CARDIOTROPHIN 1, A CYTOKINE THAT
INDUCES CARDIAC MYOCYTE HYPERTROPHY"
PROCEEDINGS OF THE NATIONAL ACADEMY
OF SCIENCES OF USA, NATIONAL ACADEMY OF
SCIENCE. WASHINGTON, US, vol. 92, no. 4,
February 1995 (1995-02), pages 1142-1146,
XP001008825 ISSN: 0027-8424

- ITESCU SILVIU ET AL: "New directions in
strategies using cell therapy for heart disease."
JOURNAL OF MOLECULAR MEDICINE (BERLIN),
vol. 81, no. 5, May 2003 (2003-05), pages 288-296,
XP002297235 ISSN: 0946-2716

- WATT D J ET AL: "SKELETAL MUSCLE STEM
CELLS: FUNCTION AND POTENTIAL ROLE IN
THERAPY" STEM CELLS, ALPHAMED PRESS,
DAYTON, OH, US, 1997, pages 75-98,
XP000989508 ISSN: 1066-5099

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

**FIELD OF THE INVENTION**

The present invention pertains to the field of stem cell therapeutics and in particular to methods of modulating stem cell proliferation.

**BACKGROUND OF THE INVENTION**

Stem cells are undifferentiated, or immature, cells that are capable of giving rise to multiple, specialised cell types and ultimately to terminally differentiated cells. Unlike any other cells, they are able to renew themselves such that essentially an endless supply of mature cell types can be generated when needed. Due to this capacity for self-renewal, stem cells are therapeutically useful for the regeneration and repair of tissues.

Stem cells have the potential for providing benefit in a variety of clinical settings. The limitation to many potential applications has been obtaining a sufficient number of target cells and stimulating terminal differentiation of these stem cells into mature tissue specific cells.

Adult bone marrow and many other somatic tissues are believed to contain a population of pluripotent stem cells that can differentiate into cells of various phenotypes. For example, a multi-potent stem cell-like population (SP) within the adult murine heart has recently been reported (Hierlihy, A.M., et al., (2002) FEBS Letters, 530:239-243). Under circumstances of attenuated growth, these cells become activated and differentiate into cardiomyocytes. Methods of repairing or regenerating damaged myocardium by administration of somatic stem cells, derived from cardiac or other tissues, and cytokines, such as stem cell factor (SCF), granulocyte-colony stimulating factor (G-CSF), granulocyte-macrophage colony stimulating factor (GM-CSF), stromal cell-derived factor-1, steel factor, vascular endothelial growth factor, macrophage colony stimulating factor, granulocyte-macrophage stimulating factor or interleukin-3, have recently been described (U.S. Patent Application 20030054973).

Cytokines are known to play a critical role in the communication between cells within an organism and act as cellular mediators that can regulate growth and differentiation. The use of cytokines to promote differentiation of stem cells has been described. For example, U.S. Patent Application 20030027330 describes methods of producing differentiated mammalian cells or tissue from stem cells by co-culturing stem cells with developing or developed allogeneic or xenogeneic cells and optionally a cytokine, growth factor or chemokine; U.S. Patent Application 20030103951 describes a method of regenerating cardiac muscle by administration of mesenchymal stem cells, which may be genetically modified to produce proteins that are important in differentiation, such as cytokines, growth factors, myogenic factors and transcription factors, and U.S. Patent Application 20020142457 describes methods for proliferating cells having the potential to differentiate into cardiomyocytes and for regulating their differentiation into cardiomyocytes using various cytokines and transcription factors.

Cardiotrophin-1 (CT-1) is a member of the IL-6 family of cytokines and is expressed in a relatively cardiac restricted manner. The genes encoding both human and mouse CT-1 have been cloned (Pennica, D., et al., (1996) Cytokine, 8:183-189; Pennica, D., et al., (1995) Proc. Natl. Acad Sci. USA, 92:1142-1146). Originally known as cardiac hypertrophy factor (CHF), CT-1 has been shown to induce cardiac hypertrophy and the use of CT-1 and its antagonists in heart failure, arrhythmic or inotropic disorders, or peripheral neuropathies has been described (see, U.S. Patent Nos. 5,534,615; 5,571,675; 5,571,893; 5,624,806 and 5,679,545), as has its use in the diagnosis and treatment of cancer (U.S. Patent Application 20020146707). Protection of adult rat hearts from injury by administration of CT-1 prior to ischaemia has also been reported (Liao, Z., et al., (2002) Cardiovasc. Res., 53:902-919).

This background information is provided for the purpose of making known information believed by the applicant to be of possible relevance to the present invention. No admission is necessarily intended, nor should be construed, that any of the preceding information constitutes prior art against the present invention.

It is an object of the invention to overcome disadvantages of the prior art.

The above object is met by the combinations of features of the main claims, the sub-claims disclose further advantageous embodiments of the invention.

**SUMMARY OF THE INVENTION**

The present invention pertains to the field of stem cell therapeutics and in particular to methods of modulating stem cell proliferation.

According to the present invention there is provided an *ex vino* method of promoting proliferation of a population of adult stem cells comprising contacting said stem cells with a polypeptide comprising a cardiotrophin-1 amino acid sequence, a derivative or active fragment thereof retaining substantially the same activity as the wild-type protein, or a polynucleotide encoding said polypeptide.

In accordance with another aspect of the present invention, there is provided use of a polypeptide comprising a cardiotrophin-1 amino acid sequence, a derivative or active fragment thereof retaining substantially the same activity as the wild-type protein, or a polynucleotide encoding said polypeptide for the preparation of a medicament for promoting proliferation of a population of adult stem cells.

In accordance with another aspect of the present invention, there is provided a composition for use in in vivo promoting proliferation and/or differentiation of adult stem ceHs comprising,

a) a polypeptide comprising a cardiotrophin-1 amino acid sequence, a derivative or active fragment thereof retaining substantially the same activity as the wild-type protein, or a polynucleotide encoding said polypeptide, and;

b) at least one modulator of stem cell proliferation, differentiation or both selected from the group consisting of:

i) one or more Wnt polypeptides;
ii) one or more nucleotide sequences encoding one or more Wnt polypeptides;
iii) Pax 7 polypeptides;
iv) a nucleotide sequence encoding Pax 7 polypeptide;

or a combination thereof.

In accordance with another aspect of the present invention, there is provided products containing a first polypeptide comprising a cardiotrophin-1 amino acid sequence, a derivative or active fragment thereof retaining substantially the same activity as the wild-type protein, or a polynucleotide encoding said first polypeptide and one or more stem cell modulators, wherein said one or more stem cell modulators is a polypeptide or a polynucleotide encoding a polypeptide as a combined preparation for simultaneous, separate or sequential use in in vivo promoting and/or differentiating adult stem cells.

This summary of the invention does not necessarily describe all necessary features of the invention but that the invention may also reside in a sub-combination of the described features.

## BRIEF DESCRIPTION OF THE DRAWINGS

These and other features of the invention will become more apparent from the following description in which reference is made to the appended drawings wherein:

**FIGURE 1** presents a schematic representation of the adenovirus construct ad-CT-1 employed by the present invention.

**FIGURES 2A and 2B** depict FACS analysis of cardiac cell suspensions treated by saline or cardiotropin-1 injection and subsequently stained with Hoechst 33342 dye. **Figure 2C** shows a Western Blot demonstrating CT-1 adenovirus expression in infected H9C2 cells. CT-1 protein of about 24.5 kDa is shown in the blot. **Figure 2D** depicts control results obtained from FACS analysis of cardiac cells 24 hours post control injection. **Figure 2E** depicts control results of FACS analysis of cardiac cells 24 hours post adenovirus injection.

**FIGURES 3A and B** show time course results demonstrating the effects of ad-CT-1 on murine heart at t = 24, 48, 72 and 96 hours post injection of ad-CT-1.

**FIGURE 4** shows results depicting the relative number of SP cells found in AdCT-1 treated hearts during a 4 day recovery period following intracardiac injection. The data was normalized against the data obtained for the control injected hearts for each time point. The control injection consisted of an equivalent Ad vector that did not encode CT-1.

**FIGURES 5A, B and C** show results of intra-cardiac administration of ad-CT-1 on SP cells from skeletal muscle (**Figure 5a**), bone marrow (**Figure 5b**) and liver (**Figure 5c**) at time points of 24 hours, 48 hours, 72 hours and 96 hours.

**FIGURE 6** shows results of FACS analysis following control and CT-1 injections of murine hearts at 3 weeks, 4 months and 1 year time points suggesting a possible age dependent effect of CT-1 on the murine heart.

**FIGURE 7** shows results suggesting that CT-1 accelerates the differentiation of adult cardiac stem cells. Mice expressing a constitutively expressed GFP transgene were injected with CT-1. The hearts were collected 24 hours later and the isolated SP cells from those hearts were co-cultured with normal primary cardiomyocytes. The results demonstrate a robust conversion of GFP-labeled SP cells into connexin-43 positive cardiomyocytes.

**FIGURE 8** depicts an illustration of experimental procedures followed to ascertain if intracardiac injection of Ad-CT-1 could affect the repair of injured skeletal muscle.

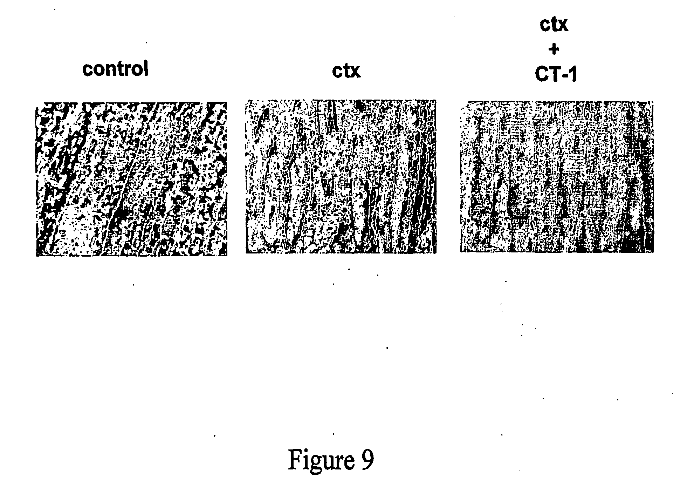

**FIGURE 9** shows results depicting the extent of damage approximately 6 days following control injection, cardiotoxin (ctx) injection, or both cardiotoxin and Ad-CT-1 injection. In these experiments, the right tibialis anterior (TA) of the hindlimb from 1 month aged mice were injected with 10 micromolar cardiotoxin. The contra-lateral leg was used as a control. In another group of mice, CT-1 was administered intracardially following cardiotoxin injection. After 6 days, the TA muscle was dissected and frozen for sectioning. The muscle sections were stained with hematoxillin/eosin to visualize membrane and structural components.

**FIGURE 10** shows results of staining muscle sections with alpha-actinin antibody. The antibody stains the sarcomeric z-bands of skeletal muscle and is an accepted indicator of muscle sarcomere integrity.

## DESCRIPTION OF PREFERRED EMBODIMENT

The following description is of a preferred embodiment by way of example only and without limitation to the combination of features necessary for carrying the invention into effect.

The present invention provides a method of modulating adult stem cell proliferation by modulating the activity of cardiotrophin (CT-1). Thus the invention provides a method of promoting adult stem cell proliferation, differentiation or both, comprising contacting the cells with CT-1, or an analogue, derivative, variant or active fragment thereof, or a compound that activates endogenous CT-1. The present invention also provides a method of inhibiting stem cell proliferation, differentiation or both, comprising contacting the cells with an inhibitor of CT-1. The method of promoting stem cell proliferation may further comprise contacting the stem cells with one or more stem cell modulators in order to promote proliferation and/or differentiation of the stem cells. In accordance, with this embodiment of the invention, the cells may be contacted concomitantly with CT-1 (or an analogue, derivative, variant or active fragment thereof, or CT-1 activator) and the one or more stem cell modulators, or the cells may be contacted sequentially.

The method may be employed to promote stem cell proliferation, stem cell differentiation or both *in vitro.* Alternatively, the method may be employed to promote stem cell proliferation, stem cell differentiation, or both *in vivo.* The present invention also contemplated methods that may be practiced *in vitro* and *in vivo.* Therapeutic applications of the present invention are not meant to be limited to diseases and disorders in which there is a need to promote stem cell proliferation and/or differentiation, for example to replace damaged or defective tissue. The method as provided herein may be employed as a therapeutic method or a preventative method. The method of the present invention also may be employed to inhibit stem cell proliferation and thus have application in the treatment of disorders characterised by inappropriate cell proliferation, such as, but not limited to cardiac hypertrophy.

*Definitions*

Unless defined otherwise, all technical and scientific terms used herein have the same meaning as commonly understood by one of ordinary skill in the art to which this invention pertains.

The term "stem cell modulator," as used herein, refers to a compound that is capable of stimulating or inhibiting stem cell proliferation, differentiation, or both proliferation and differentiation.

The term "stem cell," as used herein, refers to a cell that is capable of differentiating into one or more differentiated cell types. Stem cells may be totipotent or pluripotent cells. Totipotent stem cells typically have the capacity to develop into any cell type. Totipotent stem cells are usually embryonic in origin. Pluripotent cells are typically cells in a stem cell line capable of differentiating into several different, final differentiated cell types. Pluripotent stem cells can originate from various tissue or organ systems, including, but not limited to, blood, nerve, cardiac and skeletal muscle, skin, gut, bone, kidney, liver, pancreas, thymus, and the like.

The term "progenitor cell," as used herein, refers to a cell that is committed to a particular cell lineage and which gives rise to a particular limited range of differentiated cell types by a series of cell divisions. An example of a progenitor cell would be a myoblast, which is capable of differentiation to only one type of cell, but is itself not fully mature or fully differentiated.

The terms "proliferation" and "expansion," as used interchangeably herein with reference to cells, refer to an increase in the number of cells of the same type by cell division.

The term "differentiation," as used herein, refers to a developmental process whereby cells become specialised for a particular function, for example, where cells acquire one or more morphological characteristics and/or functions different from that of the initial cell type. The term "differentiation" includes both lineage commitment and terminal differentiation processes. Differentiation may be assessed, for example, by monitoring the presence or absence of lineage markers, using immunohistochemistry or other procedures known to a worker skilled in the art. Differentiated progeny cells derived from progenitor cells may be, but are not necessarily, related to the same germ layer or tissue as the source tissue of the stem cells. For example, neural progenitor cells and muscle progenitor cells can differentiate into hematopoietic cell lineages.

The terms "lineage commitment" and "specification," as used interchangeably herein, refer to the process a stem cell undergoes in which the stem cell gives rise to a progenitor cell committed to forming a particular limited range of differentiated cell types. Committed progenitor cells are often capable of self-renewal or cell division.

The term "terminal differentiation," as used herein, refers to the final differentiation of a cell into a mature, fully differentiated cell. For example, neural progenitor cells and muscle progenitor cells can differentiate into hematopoietic cell lineages, terminal differentiation of which leads to mature blood cells of a specific cell type. Usually, terminal differentiation is associated with withdrawal from the cell cycle and cessation of proliferation.

The term "naturally occurring," as used herein, as applied to a polypeptide or polynucleotide, refers to the fact that the polypeptide or polynucleotide can be found in nature. For example, a polypeptide or polynucleotide sequence that is present in an organism that can be isolated from a source in nature and which has not been intentionally modified by man in the laboratory is naturally-occurring,

## Methods of Modulating Stem Cell Proliferation using Cardiotrophin-1 (CT-1)

The present invention provides a method of promoting stem cell proliferation, differentiation, or both proliferation and differentiation using CT-1. CT-1 may be used alone, or in combination with one or more stem cell modulators to promote proliferation and/or differentiation of the stem cells. CT-1, and optionally, one or more stem cell modulators may be provided directly to the stem cell or they can be provided indirectly, for example, but not limited to by co-culture with cells that are capable of expressing CT-1 and/or one or more modulators. Also contemplated is the use of compounds that activate endogenous CT-1 in a stem cell, or in a cell co-cultured with a stem cell, in the methods of the invention.

The present invention further provides a method of inhibiting stem cell proliferation using one or more inhibitors of CT-1. The CT-1 inhibitor(s) can be provided directly to the stem cell or they can be provided indirectly, for example, but not limited to by co-culture with other cells that express and preferably secrete the one or more inhibitors.

Adult stem cells may be employed in the methods of the present invention. The stem cells may be pluripotent stem cells derived from a particular tissue or organ, for example, from blood, nerve, skeletal muscle, cardiac muscle, bone marrow, skin, gut, bone, kidney, liver, pancreas, thymus, and the like.

The method of the present invention is applied to adult stem cells. In another embodiment, the method of the present invention employs cardiac muscle stem cells, skeletal muscle stem cells or both.

*I. CT-1*

In accordance with the present invention, CT-1 refers to a mammalian CT-1 (also known as cardiac hypertrophy factor, or CHF). A number of cardiotrophin proteins are known in the art, for example, but not limited to mouse CT-1 (Pennica, D., et al., Proc. Natl. Acad. Sci. USA, 92:1142-1146) and human CT-1 (Pennica, D., et al., (1996) Cytokine, 8:183-189).

CT-1 may be provided as a polypeptide or as a polynucleotide encoding, and capable of expressing, the polypeptide. The sequences of various CT-1 proteins are known in the art and can be used as a basis for the preparation of CT-1 polypeptides (for example, those provided by the above references and GenBank Accession Nos AAC52173 and NP_031821 (mouse); AAD12173 and AAA85229 (human)).

The present invention also contemplates polypeptide analogues, derivatives and variants of the naturally occurring (wild-type) form of CT-1 as well as active peptide fragments of CT-1, and analogues, variants and peptidomimetic forms of the peptide fragments.

Active fragments are fragments of the naturally occurring (or wild-type) protein that retain substantially the same activity as the wild-type protein. Fragments typically are at least about 20 amino acids long. In one embodiment of the present invention, the fragments are at least about 50 amino acids long. In another embodiment, the fragments are at least about 70 amino acids long. In a further embodiment, the fragments are at least about 100 amino acids long. In still another embodiment, the fragments are at least about 150 amino acids long. The term "fragment" also encompasses polypeptides corresponding to the wild-type protein that contain a deletion of 1 to about 50 amino acids from the N-terminus, C-terminus or both the N- and C-termini of the wild-type sequence. Candidate fragments can be selected from random fragments generated from the naturally occurring protein or can be specifically designed. The activity of the fragments is tested and compared to that of the wild-type protein and those fragments with substantially the same activity as the naturally occurring protein are selected. Methods for generating polypeptide fragments are well known in the art and include enzymatic, chemical or mechanical cleavage of the wild-type protein or a recombinant version thereof, expression of nucleic acids encoding such fragments, and the like.

As is known in the art, analogues and derivatives of polypeptides, and peptidomimetic compounds may have significant advantages over the naturally occurring forms, including, for example, greater chemical stability, increased resistance to proteolytic degradation, enhanced pharmacological properties (such as, half-life, absorption, potency and efficacy), altered specificity (for example, a broad-spectrum of biological activities) and/or reduced antigenicity.

A "derivative" is a polypeptide or peptide containing additional chemical or biochemical moieties not normally a part of a naturally occurring sequence. Derivatives include polypeptides and peptides in which the amino-terminus and/or the carboxy-terminus and/or one or more amino acid side chain has been derivatised with a suitable chemical substituent group, as well as cyclic, dual and multimeric polypeptides and peptides, polypeptides and peptides fused to other proteins or carriers, glycosylated or phosphorylated polypeptides and peptides, polypeptides and peptides conjugated to lipophilic moieties (for example, caproyl, lauryl, stearoyl moieties) and polypeptides and peptides conjugated to an antibody or other biological ligand.

Examples of chemical substituent groups that may be used to derivatise polypeptides and peptides include, but are not limited to, alkyl, cycloalkyl and aryl groups; acyl groups, including alkanoyl and aroyl groups; esters; amides; halogens; hydroxyls; carbamyls, and the like. The substituent group may also be a blocking group such as Fmoc (fluorenylmethyl-O-CO-), carbobenzoxy (benzyl-O-CO-), monomethoxysuccinyl, naphthyl-NH-CO-, acetylamino-caproyl and adamantyl-NH-CO-. Other derivatives include C-terminal hydroxymethyl derivatives, 0-modified derivatives (for example, C-terminal hydroxyethyl benzyl ether) and N-terminally modified derivatives including substituted amides such as alkylamides and hydrazides.

The term "cyclic" polypeptide or peptide refers to a cyclic derivative of a polypeptide or peptide to which, for example, two or more additional amino acid residues suitable for cyclisation have been added. These additional amino acids may be added at the carboxyl terminus and at the amino terminus, or they may be at internal positions. Alternatively, a cyclic polypeptide/peptide may take advantage of cysteine residues that occur naturally in the amino acid sequence to form a disulphide bond and thereby cyclise the polypeptide/peptide. A cyclic polypeptide/peptide can contain either an intramolecular disulphide bond, *i.e.*, -S-S-; an intramolecular amide bond between the two added residues, *i.e.*, -CONH- or -NHCO-; or intramolecular S-alkyl bonds, *i.e.*, -S-$(CH_2)$-CONH- or NH-CO$(CH_2)_n$ S-, wherein n is 1, 2, or more.

Cyclic polypeptides/peptides containing an intramolecular disulphide bond may be prepared by conventional solid phase synthesis while incorporating suitable S-protected cysteine or homocysteine residues at the positions selected for cyclisation (see, for example, Sahm et al., 1996, J. Pharm. Pharmacol. 48:197). Following completion of the chain assembly, cyclisation can be performed either by selective removal of the S-protecting groups with a consequent on-support oxidation of free corresponding SH-functions, to form S--S bonds, followed by conventional removal of the product from the support and appropriate purification procedure, or by removal of the polypeptide/peptide from the support along with complete side-chain deprotection, followed by oxidation of the free SH-functions in highly dilute aqueous solution. Similarly, cyclic derivatives containing an intramolecular amide bond may be prepared by conventional solid phase synthesis while incorporating suitable amino and carboxyl side-chain protected amino acid derivatives at the positions selected for cyclisation, and cyclic polypeptides/peptides containing intramolecular -S-alkyl bonds can be prepared by conventional solid phase synthesis while incorporating an amino acid residue with a suitable amino-protected side chain, and a suitable S-protected cysteine or homocysteine residue at the positions selected for cyclisation.

A dual polypeptide/peptide consists of two of the same, or two different, polypeptides/peptides covalently linked to one another, either directly or through a spacer such as a short stretch of alanine residues or a putative site for proteolysis (see, for example, U.S. Patent No. 5,126,249 and European Patent No. 495,049). Multimers are polymeric molecules formed from a number of the same or different polypeptides/peptides or derivatives thereof. The polymerisation is carried out with a suitable polymerisation agent, such as 0.1 % glutaraldehyde (see, for example, Audibert et al., 1981, Nature 289:593).

An "analogue" is a polypeptide/peptide comprising one or more non-naturally occurring amino acid. For example, a polypeptide/peptide analogue of the invention may have one or more amino acid residues replaced by the corresponding D-amino acid residue or with another non-naturally occurring amino acid. Examples of non-naturally occurring amino acids include, but are not limited to, N-α-methyl amino acids, C-α-methyl amino acids, (β-methyl amino acids, (β-alanine ((β-Ala), norvaline (Nva), norleucine (Nle), 4-aminobutyric acid (y-Abu), 2-aminoisobutyric acid (Aib), 6-aminohexanoic acid (∈-Ahx), ornithine (orn), hydroxyproline (Hyp), sarcosine, citrulline, cysteic acid, cyclohexylalanine, α-amino isobutyric acid, t-butylglycine, tbutylalanine, 3-aminopropionic acid, 2,3-diaminopropionic acid (2,3-diaP), phenylglycine, 2-naphthylalanine (2-Nal), 1,2,3,4-tetrahydroisoquinoline-3-carboxylic acid (Tic), β-2-thienylalanine (Thi), methionine sulphoxide (MSO) and homoarginine (Har).

As is known in the art, substitution of all D-amino acids for all L-amino acids within a peptide can result in an "inverso" peptide, or in a "retro-inverso" peptide (see Goodman et al. "Perspectives in Peptide Chemistry" pp. 283-294 (1981); U.S. Patent No. 4,522,752), both of which are considered to be analogues in the context of the present invention. An "inverso" peptide is one in which all L-amino acids of a sequence have been replaced with D-amino acids, and a "retro-inverso" peptide is one in which the sequence of the amino acids has been reversed ("retro") and all L-amino acids have been replaced with D-amino acids. For example, if the parent peptide is Thr-Ala-Tyr, the retro form is Tyr-Ala-Thr, the inverso form is thr-ala-tyr, and the retro-inverso form is tyr-ala-thr (lower case letters indicate D-amino acids).

Compared to the parent peptide, a retro-inverso peptide has a reversed backbone while retaining substantially the original spatial conformation of the side chains, resulting in an isomer with a topology that closely resembles the parent peptide.

Peptidomimetics are compounds that are structurally similar to polypeptides/peptides and contain chemical moieties that mimic the function of the polypeptide or peptide of the invention. For example, if a polypeptide contains two charged chemical moieties having functional activity, a mimetic places two charged chemical moieties in a spatial orientation and constrained structure so that the charged chemical function is maintained in three-dimensional space. The term peptidomimetic thus is intended to include isosteres. The term "isostere," as used herein, refers to a chemical structure that can be substituted for a polypeptide or peptide because the steric conformation of the chemical structure is similar to that of the peptide or polypeptide, for example, the structure fits a binding site specific for the polypeptide or peptide. Examples of peptidomimetics include peptides comprising one or more backbone modifications (*i.e.,* amide bond mimetics), which are well known in the art. Examples of amide bond mimetics include, but are not limited to, $-CH_2NH-$, $-CH_2S-$, $-CH_2CH_2-$, $-CH=CH-$ (cis and trans), $-COCH_2-$, $-CH(OH)CH_2-$, and $-CH_2SO-$ (see, for example, Spatola, Vega Data Vol.1, Issue 3, (1983); Spatola, in Chemistry and Biochemistry of Amino Acids Peptides and Proteins, Weinstein, ed., Marcel Dekker, New York, p. 267 (1983); Morley, J. S., Trends Pharm. Sci. pp. 463-468 (1980); Hudson et al., Int. J. Pept. Prot. Res. 14:177-185 (1979); Spatola et al., Life Sci. 38:1243-1249 (1986); Hann, J. Chem. Soc. Perkin Trans. I307-314 (1982); Almquist et al., J. Med Chem. 23:1392-1398 (1980); Jennings-White et al., Tetrahedron Lett. 23:2533 (1982); Szelke et al., EP 45665 (1982); Holladay et al., Tetrahedron Lett. 24:4401-4404 (1983); and Hruby, Life Sci. 31:189-199 (1982)). Other examples of peptidomimetics include peptides substituted with one or more benzodiazepine molecules (see, for example, James, G. L. et al. (1993) Science 260:1937-1942) and peptides comprising backbones crosslinked to form lactams or other cyclic structures.

One skilled in the art will appreciate that not all amino acids in a peptide or polypeptide need be modified. Similarly not all amino acids need be modified in the same way. Polypeptide/peptide derivatives, analogues and peptidomimetics of the present invention thus include chimeric molecules that contain two or more chemically distinct regions, each region comprising at least one amino acid or modified version thereof.

A variant polypeptide or peptide is one in which one or more amino acid residues have been deleted, added or substituted for those that appear in the amino acid sequence of the naturally occurring protein. In the context of the present invention, a variant also retains substantially the same activity as the naturally occurring protein. Typically, when a variant contains one or more amino acid substitutions they are "conservative" substitutions. A conservative substitution involves the replacement of one amino acid residue by another residue having similar side chain properties. As is known in the art, the twenty naturally occurring amino acids can be grouped according to the physicochemical properties of their side chains. Suitable groupings include alanine, valine, leucine, isoleucine, proline, methionine, phenylalanine and tryptophan (hydrophobic side chains); glycine, serine, threonine, cysteine, tyrosine, asparagine, and glutamine (polar, uncharged side chains); aspartic acid and glutamic acid (acidic side chains) and lysine, arginine and histidine (basic side chains). Another grouping of amino acids is phenylalanine, tryptophan, and tyrosine (aromatic side chains). A conservative substitution involves the substitution of an amino acid with another amino acid from the same group.

In accordance with the present invention, an analogue, derivative, variant or active fragment has substantially the same or increased activity as compared to a naturally occurring CT-1 protein. The term "substantially identical activity" indicates an activity that is about 50% of the activity of a naturally occurring CT-1 protein. In one embodiment, substantially identical activity indicates an activity that is about 60% of the activity of a naturally occurring CT-1 protein. In another embodiment, it indicates an activity that is about 75% of the activity of a naturally occurring CT-1 protein. In still another embodiment, the analogue, derivative, variant or active fragment exhibits enhanced (increased) activity compared to a naturally occurring CT-1 protein, preferably a human CT-1 protein. Activity of CT-1 can be determined by, for example, measuring its ability to promote stem cell proliferation. In one embodiment of the invention, activity of CT-1 refers to its ability to promote cardiac stem cell proliferation. Methods of measuring increases in stem cell proliferation are known in the art and include those provided herein.

The polypeptides of the present invention can be prepared by methods known in the art, such as purification from cell extracts or the use of recombinant techniques. Shorter sequences can also be chemically synthesised by methods known in the art including, but not limited to, exclusive solid phase synthesis, partial solid phase synthesis, fragment condensation or classical solution synthesis (Merrifield (1963) J. Am. Chem. Soc. 85:2149; Merrifield (1986) Science 232:341). The polypeptides of the present invention can be purified using standard techniques such as chromatograph (e.g. ion exchange, affinity, and sizing column chromatography or high performance liquid chromatography), centrifugation, differential solubility, or by other techniques familiar to a worker skilled in the art.

The polypeptides can also be produced by recombinant techniques. Typically this involves transformation (including transfection, transduction, or infection) of a suitable host cell with an expression vector comprising a polynucleotide encoding the protein or polypeptide. The nucleic acid sequences for various CT-1 genes are known in the art (see for example, Pennica et al. Ibid., U.S. Patent Nos. 5,723,585; 5,679,545, 5,627,073 (mouse) and GenBank Accession Nos. Q16619 and NM_001330 (human)) and may be used as a basis for the polynucleotides of the invention.

The polynucleotides can be derived or purified from a suitable source by standard techniques. The polynucleotides can be genomic DNA or RNA or they can be cDNA prepared from isolated mRNA. Alternatively, the known sequences may be used to prepare probes to obtain other nucleic acid sequences encoding a CT-1 polypeptide from various sources using standard techniques. Suitable sources for obtaining the nucleic acids are those cells which are known to express the proteins of the invention, such as cardiomyocytes, as well as skeletal muscle tissue and other tissues with measurable CT-1 transcripts.

Polynucleotides encoding fragments or variants of the naturally occurring CT-1 proteins can be constructed by deletion, addition, and/or substitution of one or more nucleotides within the coding sequence using standard techniques, such as site-directed mutagenesis techniques.

The polypeptides and peptides of the present invention can also be produced as fusion proteins. One use of such fusion proteins is to improve the purification or detection of the polypeptide or peptide. For example, a polypeptide or peptide can be fused to an immunoglobulin Fc domain and the resultant fusion protein can be readily purified using a protein A column. Other examples of fusion proteins include polypeptides or peptides fused to histidine tags (allowing for purification on Nie+ resin columns), to glutathione-S-transferase (allowing purification on glutathione columns) or to biotin (allowing purification on streptavidin columns or with streptavidin labelled magnetic beads). Once the fusion protein has been purified, the tag may be removed by site-specific cleavage using chemical or enzymatic methods known in the art.

Specific initiation signals may be required for efficient translation of cloned polynucleotide. These signals include the ATG initiation codon and adjacent sequences. In cases where an entire wild-type gene or cDNA, including its own initiation codon and adjacent sequences, is inserted into the appropriate expression vector, additional translational control signal may not be needled. In other cases, exogenous translational control signals, including, perhaps, the ATG initiation codon, must be provided. Furthermore, the initiation codon must be in phase with the reading frame of the desired coding sequence to ensure translation of the entire insert. The exogenous translational control signals and initiation codons can be natural or synthetic. The efficiency of expression may be enhanced by the inclusion of appropriate transcription enhancer elements and/or transcription terminators (Bittner et al. (1987) Methods in Enzymol. 153, 516).

Suitable expression vectors for use with the nucleic acid sequences of the present invention include, but are not limited to, plasmids, phagemids, viral particles and vectors, phage and the like. For insect cells, baculovirus expression vectors are suitable. For plant cells viral expression vectors (such as cauliflower mosaic virus and tobacco mosaic virus) and plasmid expression vectors (such as the Ti plasmid) are suitable. The entire expression vector, or a part thereof, can be integrated into the host cell genome. In some circumstances, it is desirable to employ an inducible expression vector as known in the art.

Those skilled in the field of molecular biology will understand that a wide variety of expression systems can be used to provide the recombinant polypeptide or peptide. The precise host cell used is not critical to the invention. The polypeptide or peptide can be produced in a prokaryotic host (e.g., *E. coli* or *B. subtilis*) or in a eukaryotic host (e.g., *Saccharomyces* or *Pichia;* mammalian cells, such as COS, NIH 3T3, CHO, BHK, 293, or HeLa cells; insect cells; or plant cells). The methods of transformation or transfection and the choice of expression vector will depend on the host system selected and can be readily determined by one skilled in the art. Transformation and transfection methods are described, for example, in Ausubel et al. (1994) Current Protocols in Molecular Biology, John Wiley & Sons, New York; and various expression vectors may be chosen from those provided, *e.g.*, in Cloning Vectors: A Laboratory Manual (Pouwels et al., 1985, Supp. 1987) and by various commercial suppliers.

In addition, a host cell may be chosen which modulates the expression of the inserted sequences, or modifies and processes the gene product in a specific, desired fashion. Such modifications (*e.g.,* glycosylation) and processing (*e.g.,* cleavage) of protein products may be important for the activity of the protein. Different host cells have characteristic and specific mechanisms for the post-translational processing and modification of proteins and gene products. Appropriate cell lines or host systems can be chosen by one skilled in the art to ensure the correct modification and processing of the expressed heterologous protein.

The host cells harbouring the expression vehicle can be cultured in conventional nutrient media adapted as needed for activation of a chosen gene, repression of a chosen gene, selection of 15 transformants, or amplification of a chosen gene according to known procedures.

*II Compounds that Activate CT-1*

The present invention also contemplates methods of promoting stem cell proliferation using compounds that activate CT-1 and result in an increase of endogenous CT-1 or an increase in endogenous CT-1 activity. CT-1 activators may be polypeptides or genes encoding polypeptides that act upstream of CT-1 *in vivo* to upregulate expression or activity of CT-1, or they may be small molecule activators. CT-1 activators may act at a genetic level, for example to upregulate the expression of a gene encoding CT-1, or they may act at the protein level to increase the activity of a CT-1 polypeptide or to decrease the activity of an inhibitor of CT-1. CT-1 activators can be, for example, polypeptides and peptides (or analogues, derivatives, variants or peptidomimetic compounds corresponding to polypeptides, as described above), polynucleotides, oligonucleotides, antibodies or antibody fragments, or organic or inorganic small molecules.

Various screening methods known in the art can be employed to identify candidate activators. For example, activators that up- or down-regulate a target gene can be identified by monitoring cells treated with the candidate activator for an increase or decrease in the expression of the target gene. Methods such as Northern blot analysis, quantitative RT-PCR or microarray analysis can be used for this purpose. Alternatively, an increase or decrease in the corresponding protein level can be monitored, for example, by Western blot analysis.

For polypeptide or peptide activators (or analogues, derivatives, variants or peptidomimetic compounds corresponding to the polypeptides) that bind a specific protein, the binding ability can be determined using one of a variety of binding assays known in the art (see, for example, Coligan et al., (eds.) Current Protocols in Protein Science, J. Wiley & Sons, New York, NY).

For antibody or antibody fragment activators, various immunoassays can be used. Numerous protocols for competitive binding or immunoradiometric assays using either polyclonal or monoclonal antibodies with established specificities are well known in the art. Such immunoassays typically involve the measurement of complex formation between the target protein and its specific antibody. Examples of such techniques include ELISAs, radioimmunoassays (RIAs), and fluorescence activated cell sorting (FACS). Alternatively, a two-site, monoclonal-based immunoassay utilising monoclonal antibodies reactive to two non-interfering epitopes, or a competitive binding assay can be used (see, Maddox, D. E. et al. (1983) J. Exp. Med. 158:1211-1216). Such assays are well known in the art (see, for example, Hampton, R. et al. (1990) Serological Methods: A Laboratory Manual, APS Press, St Paul, Minn., Section IV; Coligan, J. E. et al. (1997, and periodic supplements) Current Protocols in Immunology, Wiley & Sons, New York, N.Y.; Maddox, D. E. et al. (1983) J. Exp. Med. 158:1211-1216).

*III CT-1 Inhibitors*

The present invention also contemplates methods of inhibiting stem cell proliferation using compounds that inhibit CT-1 and result in a decrease of endogenous CT-1 or a decrease in endogenous CT-1 activity. CT-1 inhibitors may be polypeptides or genes encoding polypeptides that act upstream of CT-1 *in vivo* to upregulate expression or activity of CT-1, or they may be small molecule inhibitors. CT-1 inhibitors may act at a genetic level, for example to downregulate the expression of a gene encoding CT-1, or they may act at the protein level to decrease the activity of a CT-1 polypeptide. CT-1 inhibitors can be, for example, polypeptides and peptides, including inactive fragments of CT-1 that interfere with the activity of the wild type protein (or analogues, derivatives, variants or peptidomimetic compounds corresponding to polypeptides, as described above), polynucleotides, oligonucleotides, antibodies or antibody fragments, or organic or inorganic small molecules.

Various screening methods known in the art as described above for identification of CT-1 activators can also be employed to identify candidate inhibitors.

*IV Stem Cell Modulators*

Also contemplated by the methods of the present invention is the use of one or more stem cell modulators(s) in addition to CT-1 (or a CT-1 activator). The stem cell modulator may promote stem cell proliferation and/or differentiation. Thus a modulator can be used to augment the activity of CT-1 and increase proliferation in a population of stem cells or it may be used to promote differentiation in a population of stem cells that have been previously expanded by treatment with CT-1. Modulators that promote stem cell differentiation may stimulate lineage commitment of the cells, or they may stimulate terminal differentiation of committed progenitor cells.

Examples of modulators that may be used in the methods of the invention include, but are not limited to, members of the Wnt family of polypeptides (including Wnt 1, Wnt 2, Wnt 3, Wnt 4, Wnt 5a, Wnt 5b, Wnt 7a and Wnt 7b, and mouse Wnt 1, Wnt 2, Wnt 3a, Wnt 3b, Wnt 4, Wnt 5a, Wnt 5b, Wnt 6, Wnt 7a, Wnt 7b, Wnt 8a, Wnt 8b, Wnt 10a, Wnt 10b, Wnt 11 and Wnt 12) and Pax7. One or more transcription factors, such as, but not limited to Pax 7, NKX2.5, GATA 4, 5 or 6, MEF2C, Hand1 and Hand2 (for cardiac muscle stem cells) and MyoD, myogenin, MRF4 and Myf5 (for skeletal muscle stem cells), may also be used, alone or in combination with a Wnt, Pax7, or both to promote terminal differentiation.

The modulators may be provided as full-length polypeptides or as active fragments or variants thereof (as discussed above), or they may be provided as polynucleotides that encode and are capable of expressing the full-length polypeptide, active fragment or variant. The amino acid and nucleic acid sequences of various Wnt proteins, Pax7 and a number of transcription factors are known in the art (for example, GenBank Accession Nos. NM_002584 and NP_002575 (Pax7)).

In one embodiment of the present invention, the modulator promotes stem cell differentiation. In another embodiment, the stem cell modulator is a Wnt polypeptide or a polynucleotide encoding a Wnt polypeptide. In a further embodiment, the stem cell modulator is a Wnt 11 polypeptide or a polynucleotide encoding a Wnt 11 polypeptide. In still another embodiment, the stem cell modulator is a Pax7 polypeptide or a polynucleotide encoding a Pax7 polypeptide.

**Testing Stem Cell Proliferation**

The ability of CT-1, analogues, derivatives, variants and active fragments thereof, or CT-1 activators, alone or in combination with stem cell modulators, to promote stem cell proliferation can be tested *in vitro* or *in vivo* using standard techniques including, but not limited to, those described herein. Inhibition of stem cell proliferation by one more CT-1 inhibitors can also be measured *in vitro* or *in vivo*.

*I.* In vitro *Testing*

Typically, stem cells are cultured in the presence and absence of the test compound(s) and at least one indicator of proliferation is subsequently monitored in the cells to determine whether proliferation has been stimulated or inhibited in those cells exposed to the test compound(s). Alternatively, a population of stem cells can be co-cultured with "educator" cells, the educator cells are exposed to the test compound(s) and at least one indicator of proliferation is subsequently monitored in the stem cells. Educator cells may be exposed to the test compound(s) prior to, or during, co-culture. Adult stem or progenitor cells derived from a variety of tissues can be used. Examples include, but are not limited to, stem cells from cardiac or skeletal muscle, pancreatic tissue, neural tissue, liver tissue or bone marrow, haematopoietic cells, myoblasts, hepatocytes, thymocytes, cardiomyocytes, and the like. Non-human embryonic stem cells may also be used.
Methods of maintaining stem cells in culture are known in the art (see, for example, Madlambayan, G.J., et al., (2001) J. Hematother. Stem Cell Res. 10, 481-492; Hierlihy, A.M., et al., (2002) FEBS Lett. 530, 239-243; Asakura, A., et al., (2002) J Cell Biol. 159,123-134). The stem cells can be cultured alone as a monoculture or they can be co-cultured with educator cells. Additional steps may be included in the screening methods before, during, or after the culture period, such as steps to identify or isolate cell populations or otherwise contribute to the success of the assay. For example, growth factors or other compounds may be employed to isolate and expand the stem cell population. EGF and FGF have been used for this purpose with neural stem cells as described by Gritti et al (J. Neurosci. (1999) 19:3287-3297), and Bcl-2 has been used in the isolation of "muscle stem cell" populations (see U.S. Patent No. 6,337,184).
Generally, a compound is tested over a range of concentrations, typically about a 1000-fold range, and a suitable exposure protocol can be readily established by one skilled in the art. When a co-culture is used, stem cell exposure to a compound can occur before, during or after the initial exposure of the stem cells to the educator cells. Alternatively, when the test compound is a polynucleotide or a compound encoded by a polynucleotide, such as a polypeptide or peptide, the stem cells can be transfected with the nucleic acid, or an expression vector comprising the polynucleotide, using standard methods described herein and elsewhere, such that the test compound is produced endogenously. Additionally, the stem cells can be exposed to a test compound by co-culture of the stem cells with another cell line, which has been transfected with the polynucleotide, or an expression vector comprising the polynucleotide, and which expresses the test compound.
As indicated above, it is further contemplated that the stem cells may not be directly exposed to the compound. For example, an educator cell population or a third cell type can be first treated with the compound and subsequently co-cultured with the stem cells. Alternatively, the stem cells can be indirectly exposed by the addition of medium that has been conditioned by such a cell population, but which is not itself included in the co-culture. In addition, it is contemplated that the stem cells may be exposed to a compound that has been incorporated into a non-liquid medium of the culture, for example, a solid, gel or semi-solid growth support such as agar, a polymer scaffold, matrix or other construct.
Indicators of stem cell proliferation that may be monitored qualitatively or quantitatively and include, for example, changes in gross morphology, total cell number, histology, histochemistry or immunohistochemistry, or the presence, absence or relative levels of specific cellular markers (e.g. cyclin Dl, phospho-histone H1 and H3, E2F and PCNA as markers of proliferation).
Changes in morphology and/or total cell number can be monitored, for example, by FACS analysis using an appropriate dye (such as one of the Hoescht dyes), BrdU incorporation or by tritiated thymidine incorporation. The presence, absence or relative levels of cellular markers can be analysed by, for example, histochemical techniques, immunological techniques, electrophoresis, Western blot analysis, FACS analysis, flow cytometry and the like. Alternatively the presence of mRNA expressed from the gene encoding the cellular marker protein can be detected, for example, using PCR techniques, Northern blot analysis, the use of suitable oligonucleotide probes and the like.
For those cells treated with both CT-1 (or a CT-1 activator) and a stem cell modulator, one or more indicators of differentiation may also be monitored in the stem cell population after treatment with the modulator. Typically differentiation is monitored by changes in gross morphology, as described above, or by the presence of lineage-specific cellular markers, which can be analysed using a number of standard techniques as indicated above.

Suitable lineage-specific cellular markers that can be monitored are known in the art. For example, induction of differentiation of cardiac muscle stem cells can be determined by monitoring the appearance of cardiomyocyte specific markers, such as connexin-43, MEF2C and myosin heavy chain; induction of differentiation in muscle-derived stem cells can be measured by examining the cells for expression of one or more myocyte marker proteins, such as myosin heavy chain, hypophosphorylated MyoD, myogenin, Myf5 and troponin T and induction of differentiation in neural stem cells, derived as neurospheres or as SP cell fractions, can be determined by monitoring the expression of GFAP, MAP2 and β-III tubulin (see, for example, Hitoshi, S., et al., (2002) Genes & Dev. 16, 846-858).

*II.* In vivo *Testing*

Alternatively, the ability of CT-1, analogues, derivatives, variants and active fragments thereof, or a CT-1 activator, to promote stem cell proliferation and/or differentiation for example, but not limited to when used in combination with one or more stem cell modulators, may be tested *in vivo* on resident stem cell populations in an appropriate experimental animal. Similarly the ability of CT-1 inhibitors to inhibit stem cell proliferation may be tested *in vivo.* Typically, the test compound(s) are administered directly to the tissue being investigated, for example, by injection. After a suitable period of time, cells are harvested from the animal and the stem cell population is analysed as described above. If necessary, an inhibitor can be tested by contacting a resident stem cell population with a compound that stimulates proliferation as well as the CT-1 inhibitor, in order to determine the ability of the inhibitor to prevent or decrease proliferation. The compound and the inhibitor may be provided to the stem cells concomitantly, or the compound may be provided before or after the inhibitor.

In one embodiment of the present invention, the ability of the compound(s) to promote stem cell proliferation is tested *in vivo* in murine cardiac tissue. The increase in cardiac stem cell-like populations (SPs) isolated from treated mice is monitored and compared to that in control mice, which are either untreated or treated with a placebo, such as buffer or saline solution. In accordance with this embodiment of the invention, a compound is considered to promote stem cell proliferation when the SP increases by at least about 2-fold. The increase in SP is measured over a time period of at least 24 hours and more typically over a period of at least 96 hours.

The ability of CT-1, analogues, derivatives, variants and active fragments thereof, or CT-1 activators to repair damaged tissue can be tested in a suitable animal model. For example, the ability of the compound(s) to repair damaged cardiac muscle tissue can be tested by administering the compound(s) to mice with coronary artery-ligation induced cardiac damage and monitoring repair of the damaged cardiac muscle (see, Guo et al., (1999) Proc. Natl. Acad. Sci. USA, 96:11507). Similarly, the ability of the compounds to repair skeletal muscle damage can be determined in animals in which muscle damage has been induced by freeze crush or cardiotoxin administration (see Megeney et al., (1996) Genes Dev., 10:1173-1183; Asakura et al., (2000) J. Cell Biol., 159:123-134).

**Applications**

The present invention provides for methods of promoting proliferation of stem cells by contacting the cells, directly or indirectly, with CT-1, an analogue, derivative, variant or active fragment thereof, or an activator of CT-1. The present invention further provides methods of promoting the proliferation and differentiation of stem cells by contacting the cells directly or indirectly with CT-1 (or an analogue, derivative, variant or active fragment thereof, or an activator of CT-1) and one or more stem cell modulators. Methods of inhibiting stem cell proliferation comprising contacting the cells, directly or indirectly, with one or more CT-1 inhibitor. The methods provided by the present invention have a number of applications. For example, the methods can be used *in vitro* to promote proliferation of stem cells wherein the cells are destined for further *in vitro* use, for example, for research purposes. The methods can be used for maintaining stem cell cultures *in vitro* and also have potential application in the development of new *in vitro* models for drug testing.

Alternatively, the methods of promoting proliferation and/or differentiation can be used to stimulate the *ex vivo* expansion and/or differentiation of stem cells and thereby provide a population of cells suitable for transplantation or administration to a subj ect in need thereof. *Ex vivo* expansion of stem cells has therapeutic indications for treating numerous disease conditions.

The methods of promoting proliferation and/or differentiation may also be used in vivo to promote proliferation and/or differentiation of resident stem cells in tissues and thereby aid in the replacement or repair of tissue damaged as a result of the disease or disorder, or after surgery or injury.

Similarly, the methods of inhibiting proliferation using one or more CT-1 inhibitors can be used to inhibit stem cell proliferation *in vivo* and thereby prevent or minimise inappropriate cellular proliferation in tissues.

Sequential methods that promote proliferation and subsequent differentiation of stem cells are also contemplated. For example, a stem cell population may be expanded *ex vivo* by contacting the cells, directly or indirectly, with CT-1 or an activator of CT-1. The expanded population of cells is subsequently administered to a subject and treated *in vivo* with one or more stem cell modulators that promotes differentiation of the stem cells *in situ.* Alternatively, both steps may be conducted *ex vivo* prior to administration of the cells to a subject.

For *in vivo* and *ex vivo* methods, the stem cells can be autologous, allogeneic or xenogeneic.

Therapeutic applications of the methods of promoting stem cell proliferation (and optionally differentiation) typically pertain to situations where there is a need to replace lost or damaged tissue, for example, after chemotherapy or radiation therapy, after muscle injury, or in the treatment or management of diseases and disorders. For example, the methods can be used with skeletal muscle stem cells in the treatment, management or prevention of degenerative muscle disorders, muscular dystrophy, neuromuscular degenerative diseases, HIV infection and the like; with neural stem cells in the treatment, management or prevention of neurodegenerative disorders, such as Parkinson's disease and Alzheimer's disease, and with cardiac muscle cells in the treatment, management or prevention of degenerative or ischemic cardiac disease, artherosclerosis, hypertension, restenosis, angina pectoris, rheumatic heart disease, congenital cardiovascular defects, arterial inflammation and other disease of the arteries, arterioles and capillaries, in the regeneration of valves, conductive tissue or vessel smooth muscle, and in the prevention of further disease in subjects undergoing coronary artery bypass graft. Other diseases or disorders that may be treated or prevented using the methods of the present invention may include, but are not limited to degenerative liver diseases, including cirrhosis and hepatitis and diabetes.

Therapeutic applications of the methods of inhibiting stem cell proliferation typically, but not always pertain to situations where there is a need to prevent or minimise inappropriate cellular proliferation, for example in the treatment or prevention of cardiac hypertrophy.

In one embodiment of the present invention, the methods are applied to cardiac stem cells. In another embodiment, the methods are applied to adult cardiac stem cells. In still a further embodiment, which is not meant to be limiting in any manner, the methods are applied to skeletal muscle stem cells.

The present invention further provides pharmaceutical compositions comprising CT-1, an analogue, derivative, variant or active fragment thereof, an activator of CT-1 or a CT-1 inhibitor, and a pharmaceutically acceptable diluent or excipient. The pharmaceutical compositions may optionally further comprise one or more stem cell modulators, one or more stem cells, or a combination thereof. Pharmaceutical compositions and methods of preparing pharmaceutical compositions are known in the art and are described, for example, in *"Remington: The Science and Practice of Pharmacy"* (formerly "Remingtons Pharmaceutical Sciences"); Gennaro, A., Lippincott, William & Wilkins, Philadelphia, PA (2000).

Administration of the pharmaceutical compositions may be via a number of routes depending upon whether local or systemic treatment is desired and upon the area to be treated. Typically, the compositions are administered locally to the area to be treated. Administration may be topical (including ophthalmic and to mucous membranes including vaginal and rectal delivery), pulmonary (e.g. by inhalation or insufflation of powders or aerosols, including by nebulizer), intratracheal, intranasal, epidermal and transdermal, oral or parenteral. Parenteral administration includes intravenous, intraarterial, subcutaneous, intraperitoneal or intramuscular injection, for example, but not limited to intracardial injection or infusion, or intracranial, e.g. intrathecal or intraventricular administration.

The compositions of the present invention may be delivered in combination with a pharmaceutically acceptable vehicle. Preferably, such a vehicle would enhance the stability and/or delivery properties. Examples include liposomes, microparticles or microcapsules. In various embodiments of the invention, the use of such vehicles may be beneficial in achieving sustained release of the active component.

When formulated for parenteral injection, the pharmaceutical compositions are preferably used in the form of a sterile solution, containing other solutes, for example, enough saline or glucose to make the solution isotonic.

For administration by inhalation or insufflation, the pharmaceutical compositions can be formulated into an aqueous or partially aqueous solution, which can then be utilised in the form of an aerosol. For topical use, the modulators or pharmaceutical compositions comprising the modulators can be formulated as dusting powders, creams or lotions in pharmaceutically acceptable vehicles, which are applied to affected portions of the skin.

The dosage requirements for the pharmaceutical compositions vary with the particular compositions employed, the route of administration and the particular subject being treated. Dosage requirements can be determined by standard clinical techniques known to a worker skilled in the art. Treatment will generally be initiated with small dosages less than the optimum dose of each compound. Thereafter the dosage is increased until the optimum effect under the circumstances is reached. In general, the pharmaceutical compositions are administered at a concentration that will generally afford effective results without causing any harmful or deleterious side effects. Administration can be either as a single unit dose or, if desired, the dosage can be divided into convenient subunits that are administered at suitable times throughout the day.

When *ex vivo* methods of treating the stem cells are employed, the stem cells can be administered to the subject by a variety of procedures. Typically, administration of the stem cells is localised. The stem cells can be administered by injection as a cell suspension in a pharmaceutically acceptable liquid medium. Alternatively, the stem cells can be administered in a biocompatible medium which is, or becomes *in situ* a semi-solid or solid matrix. For example, the matrix may be an injectable liquid which forms a semi-solid gel at the site of tissue damage, such as matrices comprising collagen and/or its derivatives, polylactic acid or polyglycolic acid, or it may comprise one or more layers of a flexible, solid matrix that is implanted in its final form, such as impregnated fibrous matrices. Such matrices are known in the art (for example, Gelfoam available from Upjohn, Kalamazoo, Mich.) and function to hold the cells in place at the site of injury, which enhances the opportunity for the administered cells to proliferate and differentiate.

*Gene Therapy*

The present invention also contemplates administration of polynucleotides encoding CT-1 (or a variant or active fragment thereof, or an activator of CT-1) and optionally a stem cell modulator, which then express the encoded product *in vivo,* by various "gene therapy" methods know in the art. Methods of administering CT-1 are known in the art. For example, CT-1 has been used in an adenovirus to treat motomeuron degeneration (Lesbordes et al., (2003), Hum. Molec. Gen. 12,1223-1229). Gene therapy includes both *ex vivo* and *in vivo* techniques. Thus host cells can be genetically engineered *ex vivo* with a polynucleotide, with the engineered cells then being provided to a patient to be treated as described above.

Alternatively, cells can be engineered *in vivo* by administration of the polynucleotide using techniques known in the art. For example, by direct injection of a "naked" polynucleotide (Feigner and Rhodes, (1991) Nature 349:351-352; U.S. Patent No. 5,679,647) or a polynucleotide formulated in a composition with one or more other agents which facilitate uptake of the polynucleotide by the cell, such as saponins (see, for example, U.S. Patent No. 5,739,118) or cationic polyamines (see, for example, U.S. Patent No. 5,837,533); by microparticle bombardment (for example, through use of a "gene gun"; Biolistic, Dupont); by coating the polynucleotide with lipids, cell-surface receptors or transfecting agents; by encapsulation of the polynucleotide in liposomes, microparticles, or microcapsules; by administration of the polynucleotide linked to a peptide which is known to enter the nucleus; or by administration of the polynucleotide linked to a ligand subject to receptor-mediated endocytosis (see, for example, Wu and Wu, (1987) J Biol. Chem 262:4429-4432), which can be used to target cell types specifically expressing the receptor.

Alternatively, a polynucleotide-ligand complex can be formed in which the ligand comprises a fusogenic viral peptide to disrupt endosomes, allowing the polynucleotide to avoid lysosomal degradation; or the polynucleotide can be targeted for cell specific uptake and expression *in vivo* by targeting a specific receptor (see, for example, International Patent Applications WO 92/06180, WO 92/22635, WO92/20316 WO93/14188 and WO 93/20221). The present invention also contemplates the intracellular introduction of the polynucleotide and subsequent incorporation within host cell DNA for expression by homologous recombination (see, for example, Koller and Smithies (1989) Proc. Natl. Acad. Sci. USA 86:8932-8935; Zijlstra et al. (1989) Nature 342:435-438).

The polynucleotide can also be incorporated into a suitable expression vector. A number of vectors suitable for gene therapy applications are known in the art (see, for example, Viral Vectors: Basic Science and Gene Therapy, Eaton Publishing Co. (2000)).

The expression vector may be a plasmid vector. Methods of generating and purifying plasmid DNA are rapid and straightforward. In addition, plasmid DNA typically does not integrate into the genome of the host cell, but is maintained in an episomal location as a discrete entity eliminating genotoxicity issues that chromosomal integration may raise.

A variety of plasmids are now readily available commercially and include those derived from *Escherichia coli* and *Bacillus subtilis,* with many being designed particularly for use in mammalian systems. Examples of plasmids that may be used in the present invention include, but are not limited to, the eukaryotic expression vectors pRc/CMV (Invitrogen), pCR2.1 (Invitrogen), pAd/CMV and pAd/fR5/GFPq (Massie et al., (1998) Cytotechnology 28:53-64). In an exemplary embodiment, the plasmid is pRc/CMV, pRc/CMV2 (Invitrogen), pAdCMV5 (IRB-NRC), pcDNA3 (Invitrogen), pAdMLP5 (IRB-NRC), or pVAX (Invitrogen).

Alternatively, the expression vector can be a viral-based vector. Examples of viral-based vectors include, but are not limited to, those derived from replication deficient retrovirus, lentivirus, adenovirus and adeno-associated virus. Retrovirus vectors and adeno-associated virus vectors are currently the recombinant gene delivery system of choice for the transfer of exogenous genes *in vivo,* particularly into humans. These vectors provide efficient delivery of genes into cells, and the transferred polynucleotides are stably integrated into the chromosomal DNA of the host. A major prerequisite for the use of retroviruses is to ensure the safety of their use, particularly with regard to the possibility of the spread of wild-type virus in the cell population. Retroviruses, from which retroviral vectors may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumour virus. Specific retroviruses include pLJ, pZIP, pWE and pEM, which are well known to those skilled in the art.

The polynucleotide is usually incorporated into the vector under the control of a suitable promoter that allows for expression of the encoded polypeptide *in vivo.* Suitable promoters which may be employed include, but are not limited to, adenoviral promoters, such as the adenoviral major late promoter, the E1A promoter, the major late promoter (MLP) and associated leader sequences or the E3 promoter; the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTR; the histone, pol III, and (β-actin promoters; B 19 parvovirus promoter; the SV40 promoter; and human growth hormone promoters. The promoter also may be the native promoter for the gene of interest. The selection of a suitable promoter will be dependent on the vector, the host cell and the encoded protein and is considered to be within the ordinary skills of a worker in the art.

The development of specialised cell lines (termed "packaging cells") which produce only replication-defective retroviruses has increased the utility of retroviruses for gene therapy, and defective retroviruses are well characterised for use in gene transfer for gene therapy purposes (for a review see Miller, A. D. (1990) Blood 76:271). Thus, recombinant retrovirus can be constructed in which part of the retroviral coding sequence (gag, pol, env) has been replaced by subject polynucleotide and renders the retrovirus replication defective. The replication defective retrovirus is then packaged into virions that can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F. M. et al. (eds.), J. Wiley & Sons, (1989), Sections 9.10-9.14 and other standard laboratory manuals. Examples of suitable packaging virus lines for preparing both ecotropic and amphotropic retroviral systems include Crip, Cre, 2 and Am. Other examples of packaging cells include, but are not limited to, the PE501, PA317, ψ-2, ψ-AM, PA12, T19-14X, VT-19-17-H2, ψCRE, ψCRIP, GP+E-86, GP+envAml2, and DAN cell lines as described in Miller, Human Gene Therapy, Vol. 1, pgs. 5-14 (1990).

Furthermore, it has been shown that it is possible to limit the infection spectrum of retroviruses and consequently of retroviral-based vectors, by modifying the viral packaging proteins on the surface of the viral particle (see, for example PCT publications WO93/25234 and WO94/06920). For instance, strategies for the modification of the infection spectrum of retroviral vectors include: coupling antibodies specific for cell surface antigens to the viral env protein (Roux et al. (1989) PNAS 86:9079-9083; Julan et al. (1992) J. Gen Virol 73:3251-3255; and Goud et al. (1983) Virology 163:251-254); or coupling cell surface receptor ligands to the viral env proteins (Neda et al. (1991) J Biol Chem 266:14143-14146). Coupling can be in the form of the chemical cross-linking with a protein or other variety (for example, lactose to convert the env protein to an asialoglycoprotein), as well as by generating fusion proteins ((for example, single-chain antibody/env fusion proteins). This technique, while useful to limit or otherwise direct the infection to certain tissue types, can also be used to convert an ecotropic vector in to an amphotropic vector.

Moreover, use of retroviral gene delivery can be further enhanced by the use of tissue- or cell-specific transcriptional regulatory sequences which control expression of the polynucleotides contained in the vector.

Another viral vector useful in gene therapy techniques is an adenovirus-derived vector. The genome of an adenovirus can be manipulated such that it encodes and expresses a gene product of interest but is inactivated in terms of its ability to replicate in a normal lytic viral life cycle. See for example Berkner et al. (1988) BioTechniques 6:616; Rosenfeld et al. (1991) Science 252:431-434; and Rosenfeld et al. (1992) Cell 68:143-155. Suitable adenoviral vectors derived from the adenovirus strain Ad type 5 d 1324 or other strains of adenovirus (for example, Ad2, Ad3, Adz etc.) are well known to those skilled in the art. Recombinant adenoviruses can be advantageous in certain circumstances in that they can be used to infect a wide variety of cell types, including peripheral nerve cells. Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (for example, retroviral DNA). Moreover, the carrying capacity of the adenoviral genome for foreign DNA is large (up to 8 kilobases) relative to other gene delivery vectors (Berkner et al. cited supra; Haj-Ahmand and Graham (1986) J. Virol. 57:267). Most replication-defective adenoviral vectors currently in use and contemplated by the present invention are deleted for all or parts of the viral E2 and E3 genes but retain as much as 80% of the adenoviral genetic material (see, e.g., Jones et al. (1979) Cell 16:683; Berkner et al., supra; and Graham et al. in Methods in Molecular Biology, E. J. Murray, Ed. (Humana, Clifton, N.J., 1991) vol. 7. pp. 109-127).

Generation and propagation of replication-defective human adenovirus vectors requires a unique helper cell line. Helper cell lines may be derived from human cells such as human embryonic kidney cells, muscle cells, hematopoietic cells or other human mesenchymal or epithelial cells. Alternatively, the helper cells may be derived from the cells of other mammalian species that are permissive for human adenovirus, i.e. that provide, in trans, a sequence necessary to allow for replication of a replication-deficient virus. Such cells include, for example, 293 cells, Vero cells or other monkey embryonic mesenchymal or epithelial cells. The use of non-human adenovirus vectors, such as porcine or bovine adenovirus vectors is also contemplated. Selection of an appropriate viral vector and helper cell line is within the ordinary skills of a worker in the art.

In one embodiment of the present invention, the gene therapy vector is an adenovirus-derived vector.

Kits

The present invention additionally provides for therapeutic kits containing CT-1, an analogue, derivative, variant or active fragment thereof, or an activator of CT-1 and optionally one or more stem cell modulators in pharmaceutical compositions. Kits comprising one or more CT-1 inhibitors in pharmaceutical compositions are also provided. Individual components of the kit would be packaged in separate containers and, associated with such containers, can be a notice in the form prescribed by a governmental agency regulating the manufacture, use or sale of pharmaceuticals or biological products, which notice reflects approval by the agency of manufacture, use or sale for human administration.

When the components of the kit are provided in one or more liquid solutions, the liquid solution can be an aqueous solution, for example a sterile aqueous solution. In this case the container means may itself be an inhalant, syringe, pipette, eye dropper, or other such like apparatus, from which the composition may be administered to a patient.

The components of the kit may also be provided in dried or lyophilised form and the kit can additionally contain a suitable solvent for reconstitution of the lyophilised components. Irrespective of the number or type of containers, the kits of the invention also may comprise an instrument for assisting with the administration of the composition to a patient. Such an instrument may be an inhalant, syringe, pipette, forceps, measured spoon, eye dropper or any such medically approved delivery vehicle.

Results

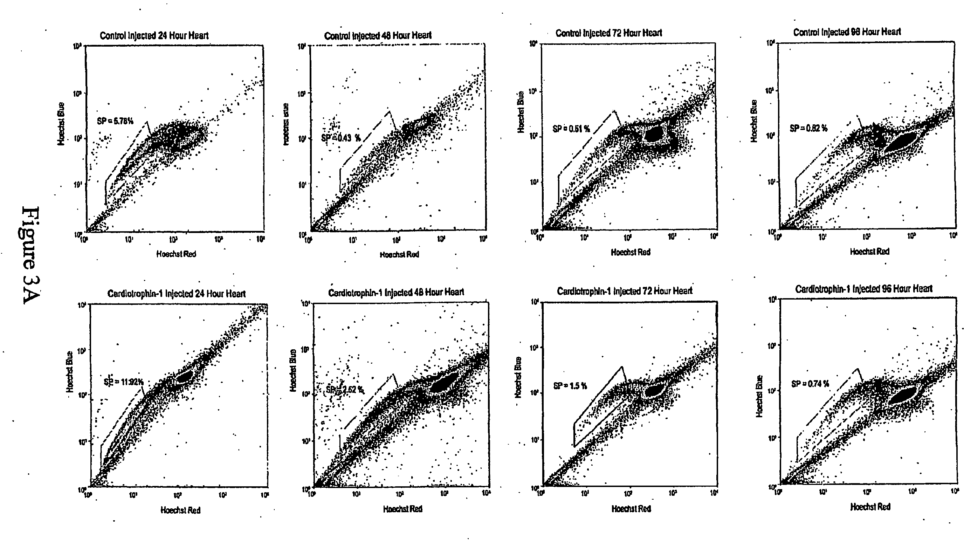

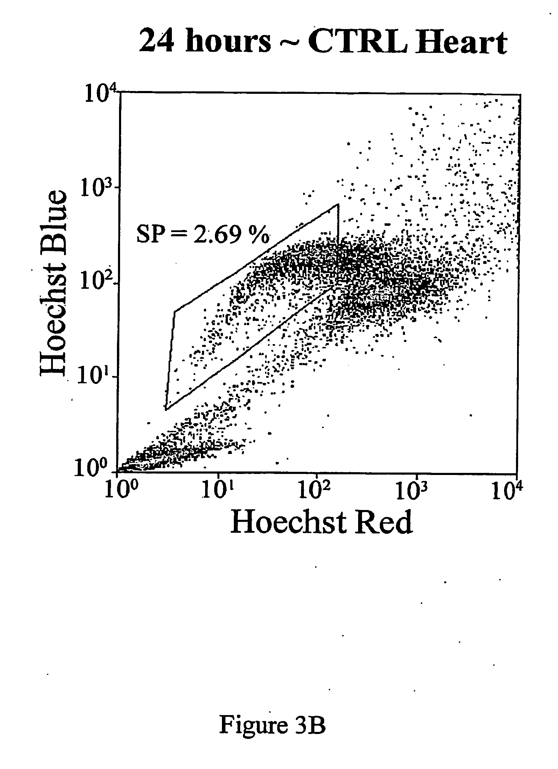

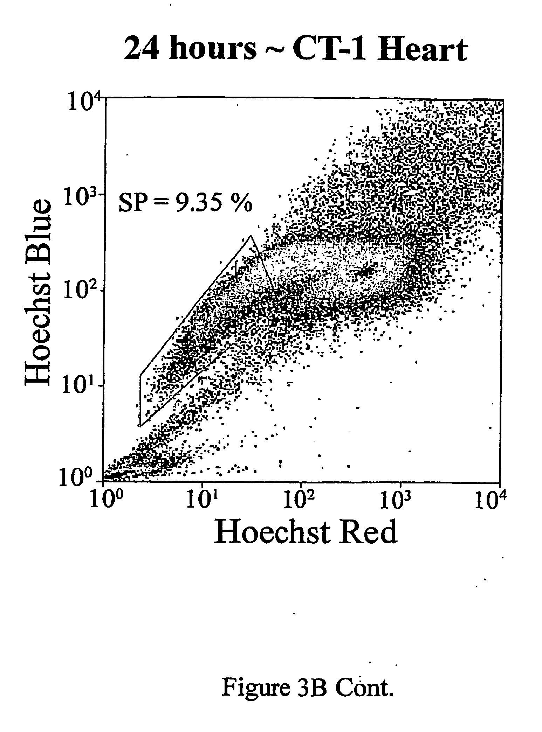

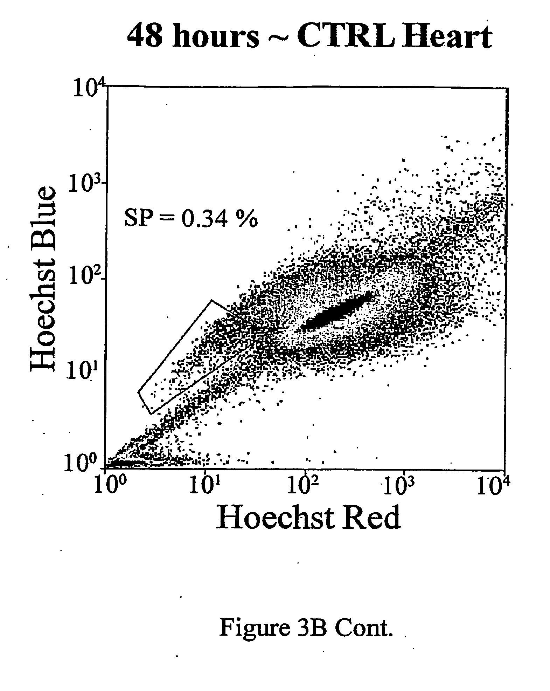

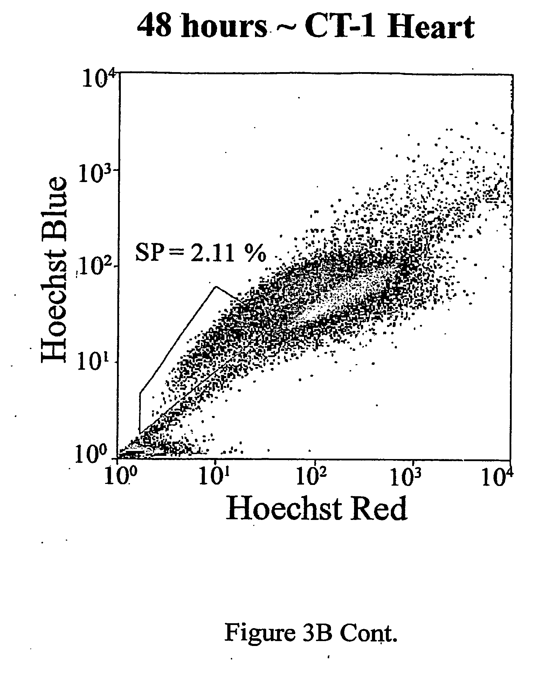

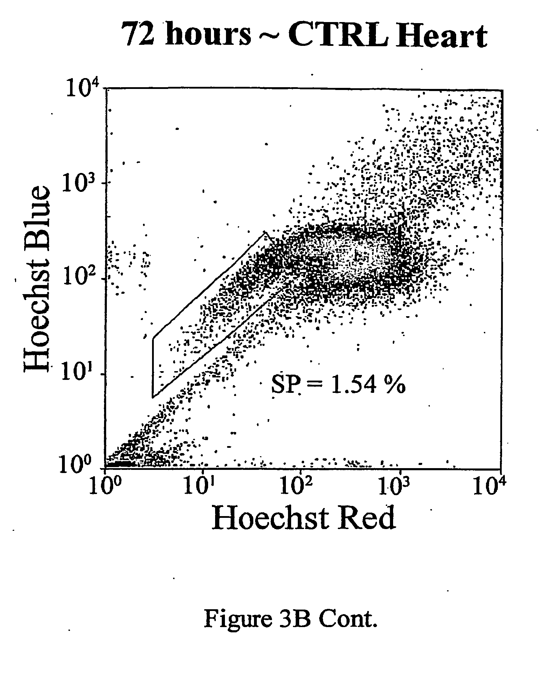

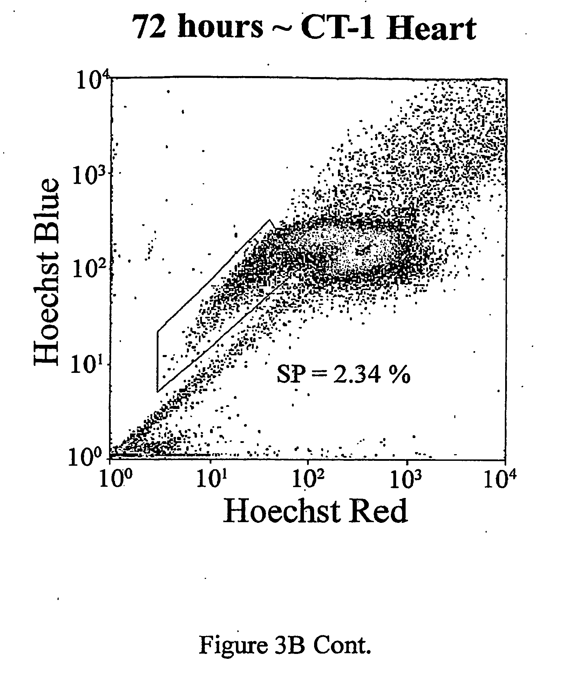

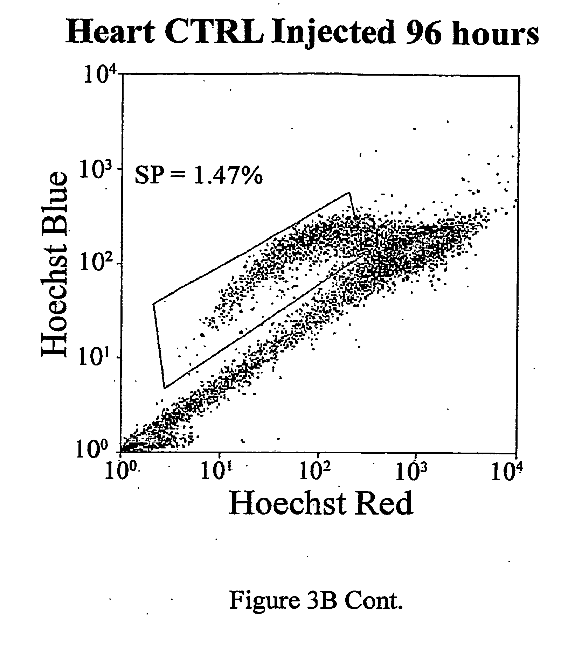

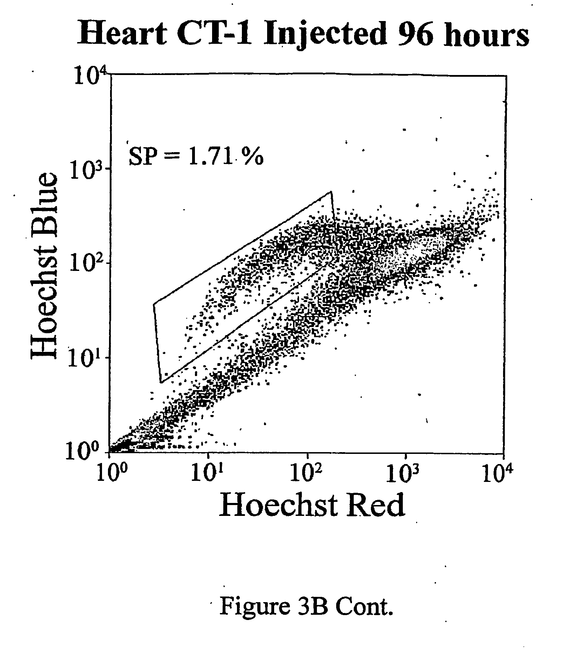

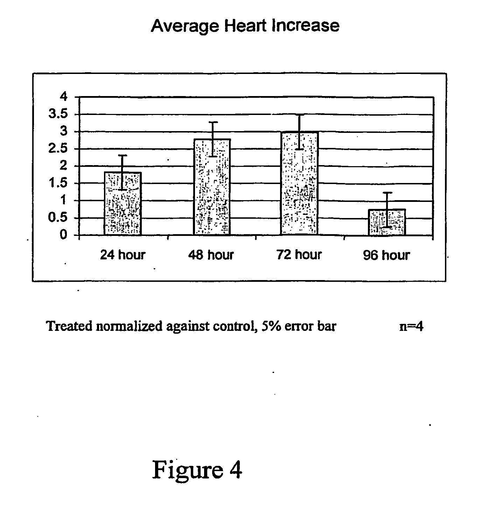

CT-1 produced and secreted from cells transformed with a suitable nucleotide construct promote proliferation and/or differentiation of stem cells. Referring now to Figures 3 and 4 there is shown time course results depicting the effects of CT-1 on murine heart cells at 24, 48, 72 and 96 hours post intracardiac injection. The data suggests that SP cells proliferate in response to CT-1 treatment. Therefore, the present invention provides a method of promoting proliferation of stem cells in a subject comprising administering a composition comprising CT-1, or a composition capable of producing CT-1 to the subject.

In an embodiment of the method as defined above, the stem cells comprise cardiac cells, more preferably cardiac SP cells. However, it is also contemplated that the stem cells may comprise stem cells derived from other fluids, tissues and or organs, for example, but not limited to,skeletal muscle stem cells, bone marrow stem cells, liver stem cells, or the like. In an alternate embodiment of the present invention, which is not meant to be limiting in any manner, the stem cells may comprise skeletal muscle stem cells. Preferably the stem cells are human stem cells.

Compositions comprising or encoding CT-1 may be administered to a subject by any route known in the art, for example, but not limited to by injection or infusion. Without wishing to be limiting in any manner, such injection routes include, but are not limited to intramuscular, subcutaneous, intraperitoneal, intravenous, intraarterial injection, or the like. In an embodiment of the present invention, the composition is administered by intramuscular injection. In a further embodiment of the present invention, the composition is administered by intracardial injection or infusion.

As discussed above, the results shown in Figures 3 and 4 indicate that CT-1 promotes proliferation of cells, for example, but not limited to stem cells in a subject treated therewith. Although cells expressing and secreting CT-1 permit about continuous administration of CT-1 to the subject, the results obtained herein indicate that there is a transient increase in a population of SP cells following continuous administration of CT-1. Further, the results indicate that the total number of SP cells increase over a period of about 24 hours to about 96 hours following treatment with CT-1 as compared to controls. These results suggest that CT-1 be administered in multiple doses to avoid periods in which stem cells are unresponsive to treatment with CT-1. Further, multiple doses of CT-1 may promote greater proliferation and/or differentiation of stem cells as compared to single or continuous doses of CT-1. Also, multiple doses spaced apart at intervals as described herein may promote greater proliferation and/or differentiation of stem cells as compared to multiple doses delivered, for example at intervals of about 24 hours.

The present invention provides a method of promoting proliferation of stem cells in a subject comprising administering two or more compositions comprising CT-1 to the subject. Preferably, the time period between the first and second doses, and optionally between any two doses of CT-1 that are administered to the subject is greater than about 24 hours, more preferably at least about 48 hours, still more preferably at least about 72 hours, and still more preferably at least about 96 hours, or more. The present invention also contemplates administering two or more doses of CT-1 to a subject, wherein the time period between any two doses is about 2 days, 3 days, 4 days, 5 days, 6 days, 7days, 10 days, 14 days, 21 days, 28 days, 1 month, 2 months, 3 months, 6 months, 1 year, or any time there in between. The two or more doses may be identical, or they may be different, for example, in amount of CT-1, the formulation, components in the composition, or any combination thereof. The present invention also contemplates administering three, four, five, six, seven or more compositions comprising CT-1 to a subject.

The present invention also provides a method of screening for compounds that increase proliferation of cells in a subject comprising,

a) administering a compound to one or more subjects belonging to a test group;

b) isolating a plurality of cells from at least one fluid, tissue or organ of the one or more subjects;

c) subjecting the plurality of cells to FACS analysis and quantifying the number of cells comprising one or more defined characteristics;

d) comparing the number of cells with the one or more defined characteristics to results obtained from one or more control subjects.

The cells may comprise a single type of cell or a combination of many types of cells. In an embodiment, the cells comprise stem cells. In an alternate embodiment the cells comprise cardiac cells, or skeletal muscle cells. In still an alternate embodiment, the cells comprise cardiac stem cells. In still an alternate embodiment, the cells comprise cardiac SP cells.

It is also contemplated that the step of subjecting (step c) may further comprise isolating the cells.

In an alternate embodiment, the step of administering (step a) may comprise administering one or more compounds, wherein at least one compound is a CT-1 polypeptide or a compound that exhibits substantially the same biological activity as a CT-1 polypeptide, and wherein the one or more compounds further comprise at least one stem cell modulator, for example, but not limited to, one or more signaling molecules, such as, but not limited to one or more Wnt polypeptides, Pax 7, or both.

In an alternate embodiment, there is provided a method of screening for compounds that increase proliferation of cells in a subject comprising,

a) isolating a plurality of cells from at least one fluid, tissue or organ of one or more test subjects and optionally purifying the plurality of cells to enrich for one or more specific cell populations;

b) treating the plurality of cells or the one or more enriched cell populations with a compound;

c) culturing the cells or cell populations for a period of time sufficient to permit the cells or cell populations to proliferate and/or differentiate, and;

d) comparing the number of colony cells resulting from the step of culturing to results obtained from one or more controls.

The step of comparing (step d) may further comprise subjecting the cells to FACS analysis to identify one or more specific populations of cells, for example, but not limited to SP cells.

The above description is not intended to limit the claimed invention in any manner, furthermore, the discussed combination of features might not be absolutely necessary for the inventive solution.

The present invention will be further illustrated in the following examples. However it is to be understood that these examples are for illustrative purposes only, and should not be used to limit the scope of the present invention in any manner.

**Examples**

**EXAMPLE 1: Cardiotrophin-1 Increases Cardiac Stem Cell-like Population (SP)**

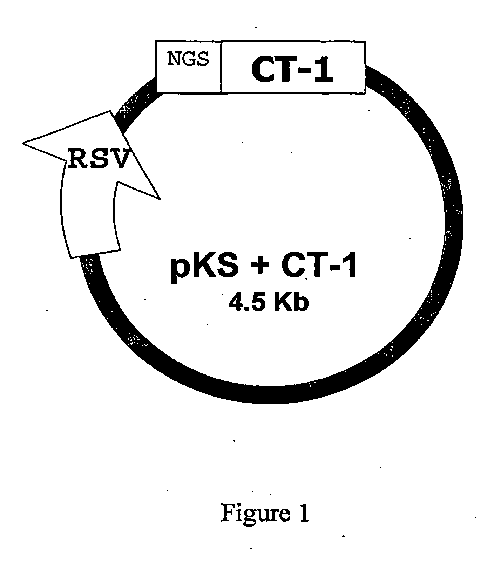

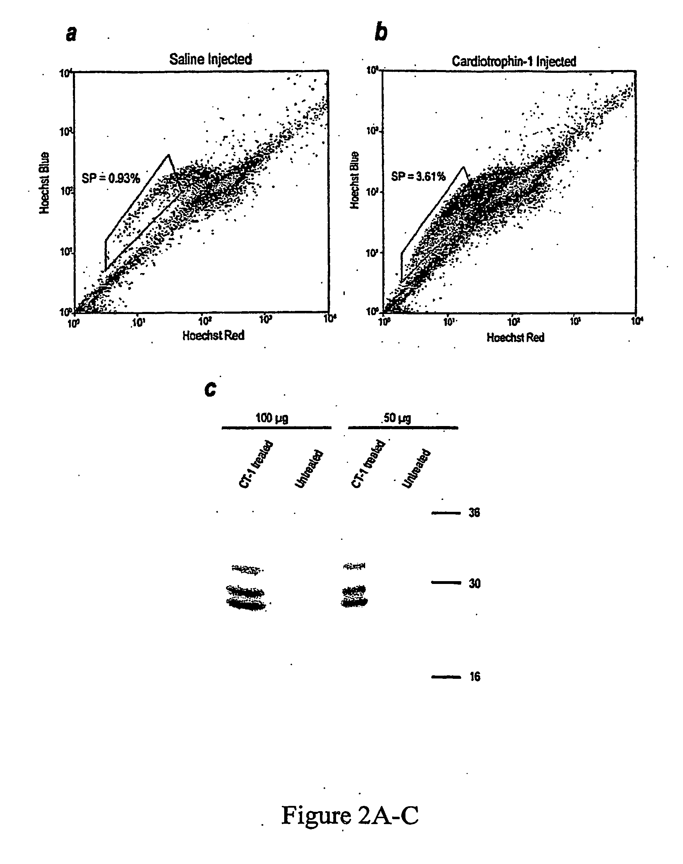

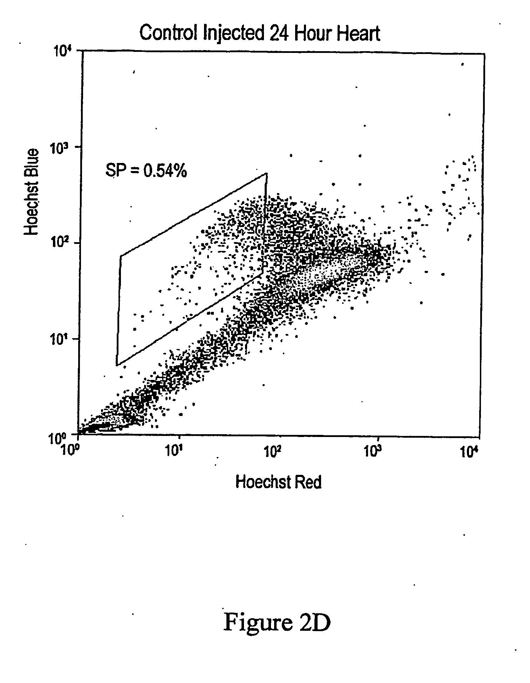

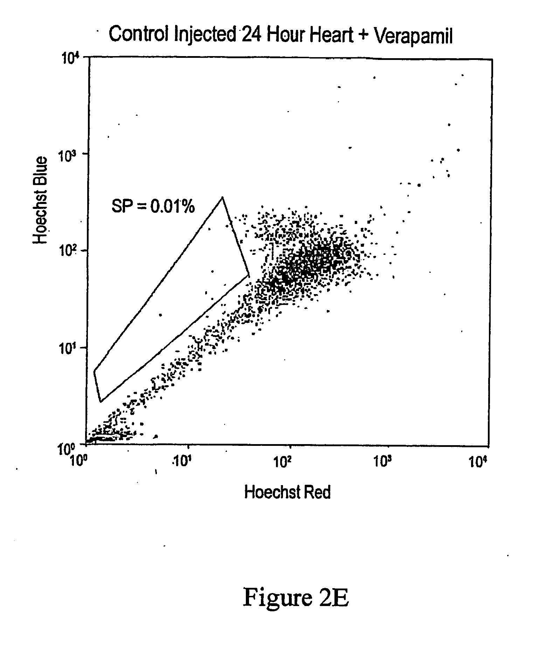

An adenovirus construct containing the full-length CT-1 driven by the ubiquitous RSV promoter was constructed (ad-CT-1) (Figure 1). CT-1 was fused to the nerve growth signal to promote secretion of CT-1 from the cell. $7.5 \times 10^7$ PFU of ad-CT-1 was injected into 2-month-old murine hearts. Parallel control experiments using 50 $\mu$L of sterile PBS were also conducted. Cells were isolated and analysed by FACS 72 hours post injection. Figure 2 depicts FACS analysis of the cardiac cell suspensions stained with Hoechst 33342 dye and analysed according to the Hoechst 33342 HSC Staining and Stem Cell Purification Protocol (see Goodell, M., et al. (1996) J Exp Med 183,1797-806, which is herein incorporated by reference). Cells were collected from three hearts. The saline injected heart exhibited an SP population of about 0.93% (Figure 2A) while the cardiotrophin injected heart exhibited a population of about 3.61 % (Figure 2B).

Figure 2C presents evidence of CT-1 adenovirus expression in infected H9C2 cells. Cells were treated with adenovirus and 72 hours later the media from these cells along with untreated control H9C2 cell media was collected for Western Blot analysis using an anti-CT-1 antibody. CT-1 protein of about 24.5 kDa is shown in the blot.

Figure 2D depicts results obtained from FACS analysis of cardiac cells 24 hours post control injection. Figure 2E depicts results of FACS analysis of cardiac cells 24 hours post control adenovirus injection. Verapamil is a drug (channel blocker) that may be used to define the SP population. For example, cells treated with verapamil exclude Hoescht dye and the SP population is no longer detected by FACS analysis. There is no change in the verapamil sensitive SP population following treatment with the control adenovirus.

A time course showing the effects of ad-CT-1 on murine heart at t = 24, 48, 72 and 96 hours is presented in Figure 3A. A second time course showing the effects of CT-1 on cardiac cells at t=24 hours, 48 hours, 72 hours and 96 hours is presented in Figure 3B. Cardiac cell suspensions were stained with Hoechst 33342 dye and subjected to FACS analysis. Prior studies using a variety of tissue sources have determined that Hoechst dye- stained cell suspensions reveal a Hoechst effluxing sub-population of cells (side population or SP cells). These cells possess stem cell-like activity, reduction or absence of differentiation markers, and are also characterized by a sensitivity to the presence of verapamil, an inhibitor of multi-drug resistance-like proteins [Goodell et al. (1997), Nat. Med. 3, 1337-1343; Jackson et al. (1999), Proc.Natl.Acad.Sci.USA 96, 14482-14486.]. Therefore, to evaluate the presence of a resident myocardial stem cell population, hearts of mature mice (~2 mos.) are enzymatically dissociated into a single cell suspension and subjected to FACS analyses. FACS was performed using a DakoCytomation MoFlo flow cytometer equipped with dual lasers. Fluorescence was measured at two wavelengths. Forward- and sidescatter was measured at 488 nm (Spectraphysic Argon laser). Hoescht dye was excited at 350 nm (I90C UV laser from Coherent). Blue emission was measured at 424nm (424/44bandpass filter) and red emission above 675 nm (675AGLP long pass filter). A 510DLP dichroic mirror was used to split these two wavelengths. FACS analyses of the myocardial cell suspensions reveal a robust Hoechst dye-excluding SP population from murine heart tissues. Total cells were isolated from 2-month-old murine hearts that received 7.5 X $10^7$ PFU of ad-CT-1 or lacZ adenovirus by direct intracardiac injection.

Referring now to Figure 4, there is shown results depicting the relative number of SP cells found in AdCT-1 treated hearts during a 4 day recovery period following intra-cardiac injection. The data was normalized against the data obtained for the control injected hearts for each time point. The data indicates that there is about a 3-fold increase in cardiac SP cells over time with large increases seen within a relatively short time period, for example, at around about 48h to about 72 hours.

Referring now to Table 2, there is shown the ratio of heart mass to body mass of subjects treated with CT-1 as compared to controls.

Table 2: Comparison of Heart Mass/Body Mass Ratios of subjects treated with CT-1

| Mouse Treatment | Body Mass (g) | Heart Mass (mg) | Ratio (heart mg /body g) |
|---|---|---|---|
| CTRL #1 | 19.65 | 144.10 | 7.333 |
| #2 | 19.96 | 117.00 | 5.862 |
| #3 | 18.06 | 127.00 | 7.032 |
| #4 | 20.94 | 119.70 | 5.716 |
| CT-1 #1 | 29.98 | 146.10 | 4.873 |
| #2 | 21.01 | 122.90 | 5.849 |
| #3 | 23.28 | 146.20 | 6.280 |
| #4 | 22.81 | 129.20 | 5.664 |

Statistical analysis indicates that no significant difference exists between the ratios obtained for the control group and the CT-1 treated group (p > 0.05). These results suggest that CT-1 may be administered to a subject to prevent or treat a variety of conditions or diseases, for example, but not limited to cardiac conditions or diseases, without promoting cardiac hypertrophy in the subjects.

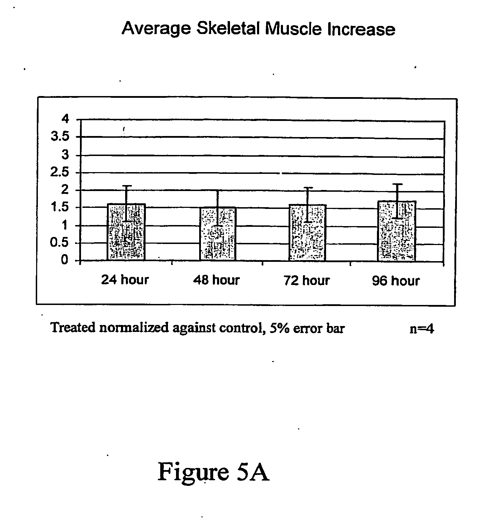

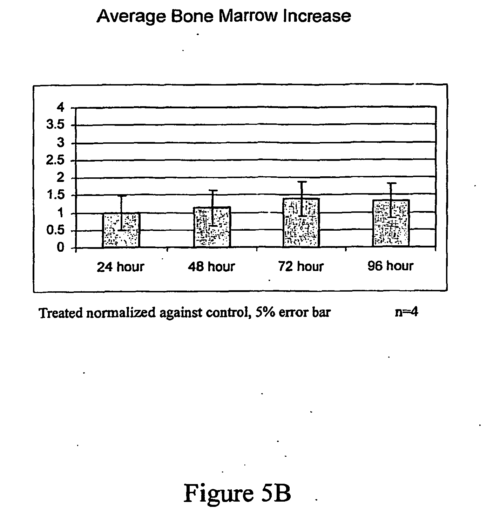

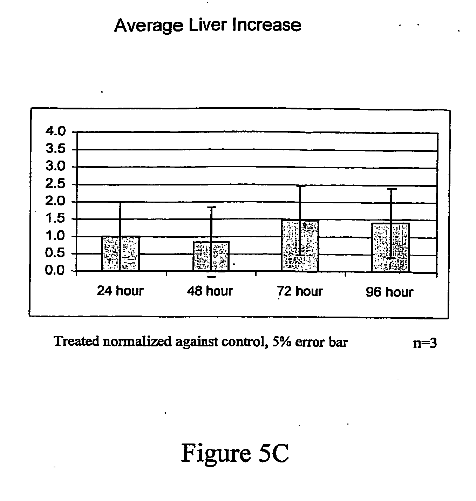

The effects of intra-cardiac administration of ad-CT-1 on skeletal muscle, bone marrow and liver at time points of 24 hours, 48 hours, 72 hours and 96 hours is shown in Figures 5A-C. The results indicate that intra-cardiac injection of ad-CT-1 provides a suitable means of selectively promoting proliferation of cardiac stem cells in a.subject. Similarly, but without wishing to be limiting in any manner, intra-muscular injection of CT-1 or a composition capable of producing CT-1 in a specific muscle of a subject may result in selective proliferation of stem cells in that muscle rather than in secondary areas such as, but not limited to the heart and other muscles or tissues. In contrast, but without wishing to be limiting, administration of CT-1 or a composition capable of producing CT-1 to a subj ect by one or more routes that permit CT-1 to contact a variety of tissues or organ systems may result in non-selective proliferation of stem cells in one or more tissues or organ systems, for example, but not limited to heart and skeletal muscle tissues. These conclusions are supported by results of Methocult® assays that employ a methylcellulose or gel like medium containing factors necessary to promote colony formation. This assay may be employed to ascertain if a population of stem cells has undergone activation at some point during their life-cycle. Briefly, the experiment was performed according to the manufacturer's instructions (StemCell Technologies, Catalog #28405, Version 3, February 2004) by plating about 10 000 cells/plate for mice injected with adCT-1 or adLacz at times = 24, 48, 72, or 96 hour post injection. Both the side population (SP) and main population (MP) fractions from heart, skeletal muscle, and bone marrow were examined. Cells were cultured for two weeks and subsequently counted for colonies and stained for various cellular markers, including hematopoietic markers. The *in vivo* administration of Ad-CT-1 vector resulted in increased proliferation of one or more skeletal muscle stem cell populations. For example, although the increase in SP in Ad-CT-1 exposed skeletal muscle was small, primary cell cultures derived from skeletal muscle and grown in permissive conditions (*i.e.* methocult medium) led to about a 10-fold increase in the number of haematopoietic colonies as compared to control treated cultures.

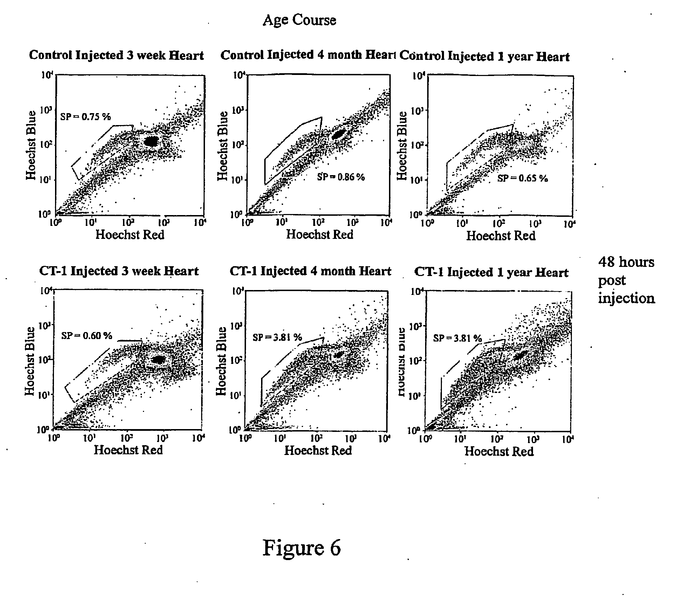

Referring now to Figure 6, there is shown results suggesting that administering of CT-1 promotes proliferation of cells in murine subjects greater than about 3 weeks of age. The present invention thus contemplates methods as defined herein and throughout, wherein the subject is older than about 3 weeks of age, preferably two months of age. In an embodiment, wherein the subject is a human, the present invention contemplates methods as defined herein and throughout wherein the subject is older than about 1 month, preferably older than about 2 months, more preferably older than about 6 months, more preferably older than about 1 year of age. In an alternate embodiment, the subject is an adult human subject.

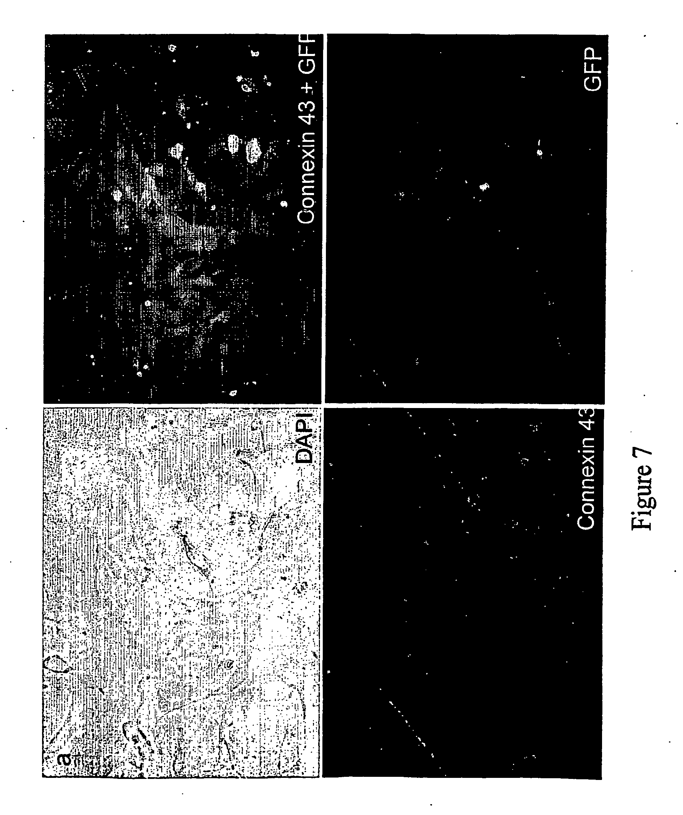

Referring now to Figure 7, there is shown results suggesting that CT-1 accelerates the differentiation of adult cardiac stem cells. Mice expressing a constitutively expressed GFP transgene were injected with CT-1. The hearts were collected 24 hours later and the isolated SP cells from those hearts were co-cultured with normal primary cardiomyocytes. The results demonstrate a robust conversion of GFP labeled SP cells into connexin-43 positive cardiomyocytes. The results provide evidence that CT-1 may be employed to promote proliferation and/or differentiation of cardiac stem cells in a subject. Further, based on the results obtained from the experiments, the present invention also provides a method of screening for compounds that modulate cardiac stem cell proliferation, differentiation or both proliferation and differentiation comprising the steps of:

a) isolating a plurality of cells comprising cardiac stem cells from a subject;
b) treating the cells with one or more compounds;
c) culturing the cells treated in step b with one or more normal primary cardiomyocytes for a period sufficient to permit said cells to undergo proliferation, differentiation or both, and;
d) measuring the number of proliferated cells, differentiated cells, or both proliferated and differentiated cells.

The step of treating may comprise contacting the cells with CT-1, or a fragment or derivative thereof, a modulator of CT-1 activity, a wnt polypeptide, or a fragment or derivative thereof that exhibits wnt activity, Pax 7, or any combination thereof. Other signal transduction molecules may also be employed. It is also contemplated that prior to the step of isolating, a subject is treated with CT-1 to promote proliferation of one or more stem cell populations. In a further embodiment, the plurality of cells comprising cardiac stem cells may comprise a marker or the like that permits them to be differentiated from normal primary cardiomyocytes. For example, the plurality of cells comprising cardiac stem cells may be GFP labelled, whereas the normal primary cardiomycytes are non GFP labelled. Any method of differentiating between the plurality of cells comprising cardiac stem cells and the normal cardiomycytes may be employed in the method as described herein.

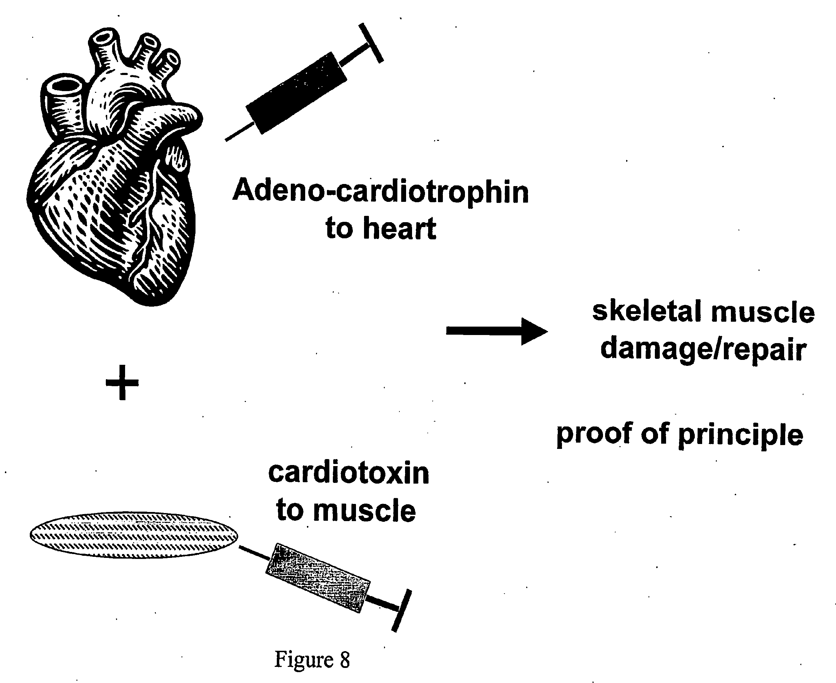

Referring now to Figure 8, there is shown an illustration generally depicting experimental procedures followed to determine whether intracardiac injection of Ad-CT-1 could affect the repair of injured skeletal muscle. The experiment was performed to explore observations made with the methocult experiments. In those experiments, it was noted that intracardial injection of CT-1 led to an increase in the number of "stem cell-like" colonies from skeletal muscle that formed on methocult media. These results suggest that CT-1 activates a stem cell-like population in skeletal muscle and that this population may contribute or enhance the skeletal muscle repair process. To corroborate these observations, damage to the murine hind-limb was induced by injecting a snake venom cardiotoxin. Cardiotoxin induces extreme muscle degeneration within a very short period of time (about 1-2 days). However, it is well known that skeletal muscle can sufficiently repair itself several days after the damage. Here, injection of CT-1 immediately after inducing cardiotoxin damage was performed. Further, similar experiments were performed wherein CT-1 was administered 1-3 days after cardiotoxin injection.

Referring now to Figure 9, there is shown results depicting the extent of damage approximately 6 days following control injection, cardiotoxin (ctx) injection, or both cardiotoxin and Ad-CT-1 injection. In these experiments, the right tibialis anterior (TA) of the hindlimb from 1 month aged mice was injected with 10 micromolar cardiotoxin. The contra-lateral leg was used as a control. In another group of mice, CT-1 was administered intracardially following cardiotoxin injection. After 6 days, the TA muscle was dissected and frozen for sectioning. The muscle sections were stained with hematoxillin/eosin to visualize membrane and structural components. More muscle integrity is noted when CT-1 is administered at about the same time as cardiotoxin.

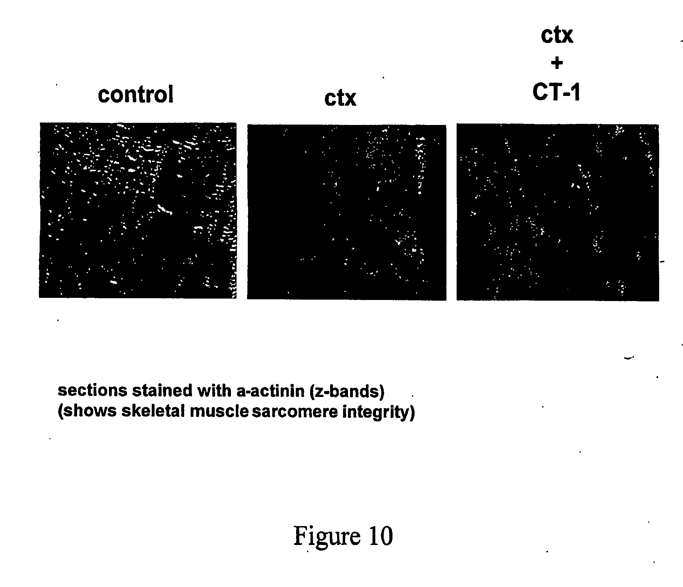

Referring now to Figure 10, there is shown muscle cross-sections treated as described above, and stained with the antibody alpha-actinin. Alpha-actinin stains the sarcomeric z-bands of skeletal muscle and is an accepted indicator of muscle sarcomere integrity. The results suggest that the muscle treated with CT-1 exhibits better alpha-actinin staining than the muscle treated with cardiotoxin (ctx) alone. Without wishing to be bound by theory or limiting in any manner, these results suggest that CT-1 may be employed to enhance skeletal muscle recovery following injury and/or CT-1 may protect muscle from degeneration following injury.

**General Protocols**

Bone Marrow Isolation

Remove the leg from the mouse. Remove the long bone in the upper leg of the mouse (femur), may also isolate the lower leg to increase bone marrow cell numbers.

1) Remove surrounding muscle and other tissue
2) Cut bone as high as possible
3) Remove tissues surrounding the knee and cut off long bone
4) Once bone is free use an insulin syringe to flush out bone marrow into a 5cm tissue culture dish.
5) Use 5 mL of PBS to flush out bone, be sure to flush out both ends.
6) Repeat as required.
7) Once bone marrow is collected, triturate in dish with 18 gauge needle.
8) Move cells to a 15 mL Falcon tube. Keep on ice.
9) For FACS continue on with protocol from point at which you spin down cells and then wash with DMEM+ *no digestion is required*

Antibody Staining for FACS Protocol

1) Follow HOECST protocol up until final wash, just prior to re-suspension in HBSS+.
2) Re-suspend in PBS + 2% FBS in 1 ml/heart.
3) Count cells using hemocytometer

$$\text{Formula} = \text{\# cells/mm}^3 \text{ X dilution X 10 000 (\#cells/mL)}$$

4) To do saturation curve:

> Dilute cells using PBS + 2%FBS to a final concentration of 3 000 000 cells/mL
> Add 100 $\mu$L to each of 8 tubes (two control and six with various antibody concentrations)
> Next dilute antibody solution (ex: Make 400 $\mu$L at 5 $\mu$g/mL where the initial concentration is 100 $\mu$g/mL) 5(400)=100($x$) x=20 $\mu$L
Put 20 $\mu$L of antibody into 380 $\mu$L of dH$_2$O
> Take 100 $\mu$L and add to cells. Call this control #1 which contains NO 2° antibody.
> Take another 100 $\mu$L and add to cells and add 2° antibody.
> With the remaining antibody (200 $\mu$L) add 200 $\mu$L of dH$_2$O. This yields 400 $\mu$L at 2.5 $\mu$g/mL.
> Continue until you have completed the appropriate numbers of dilutions.
> Control #2 will be the cells without 1 ° antibody.

5) Incubate cells with primary antibody for 15 minutes on ICE.
6) Spin cells and remove supernatant.
7) Wash cells with PBS + 2% FBS.
8) Re-suspend in 200 $\mu$L of PBS + 2% FBS.
9) Add secondary antibody and incubate for 15 minutes on ICE.
10) Wash cells again with PBS + 2% FBS.
11) Re-suspend in 500 $\mu$L of HBSS +.
12) Move into small round bottomed falcon tubes *for FACS*
13) Use 1 cc insulin syringe to remove any clumps of cells
14) Remember to bring a 15 mL tube with 1 mL of media, to be used to collect cells.
15) perform FACS

RECIPES FOR FACS

VERAPAMIL (100X)

Make a final stock solution of 100X
Suspend with 95% ethanol
For a final of 50 $\mu$M

HOECHST 33342 (200X)

Make a final stock solution of 200X
Suspend with water
For final of 1 mg/mL

DMEM +

388 mL DMEM
8 mL FBS
4 mL of 1 M Hepes

HBSS+

100 mL HBSS (Hanks Balanced Salt Solution)
2mL FBS
1 mL of 1 M Hepes

COLLAGENASE-DISPASE

Make in the hood
Dissolve entire 500mg collagenase bottle with 5 mL of IX PBS
For each 5 mL aliquot of collagenase-dispase solution add:

> 4.34 mL dispase 2 (t.c. freezer)
> 500 $\mu$L of suspended collagenase (+4°C fridge)
> 125 $\mu$L of 100 mM CaCl$_2$

PBS + 2% FBS *for use when antibody staining*

500 mL PBS
10 mL FBS

Hoechst 33342 Stem Cell staining and Purification Protocol

1) Turn on hood and thaw collagenase/dispase in 37°C water bath.

2) Kill mice, spray with ethanol and dissect out heart.

3) Put heart into cold PBS (regular PBS stored at 4°C)

4) Using tweezers, squeeze out excess blood from the hearts

5) Store in cold PBS until ready to digest

6) In the hood, with two blades, mice up hearts. (ensure hearts are moist - if they get pastey add 100 $\mu$L PBS)

7) Then move minced hearts to a small tissue culture dish

8) Add 4-5 mL per 1 to 4 hearts of collagenase/dispase

9) Pipet up and down to mix well and split up clumps of cells

10) Put into incubator at 37°C for 35 - 38 minutes

11) Pass through a 75 micron mesh filter (from netwell plate)

12) Rinse the filter with 5 mL of PBS and collect in 50 mL tube.

13) Transfer contents of 50 mL tube to a 15 mL tube.

14) Spin for 10 minutes at 1.2 setting, remove supernatant, and re-suspent in 8 mL of cold DMEM + and spin again for 10 minutes at 1.2 setting.

15) Carefully remove the supernatant and resuspend the pellet in either, 2 mL DMEM + per heart (estimate the number of cells)

16) Stain with Hoechst and Verapamil (both stored in the freezer)

add 5 $\mu$L/mL of 200X Hoechst to all of the cells

take 1 mL of Hoechst stained cells and put into a new 15 mL tube

add 15 $\mu$L/mL of 100X verapimil

17) Let sit in incubator at 37°C for 90 minutes. Ensure temperature is constant.

18) Put on ice for 5 minutes.

19) Spin for 10 minutes at 1.2 setting, remove supernatant and resuspend in 8 mL of cold DMEM + and spin again for 10 minutes at 1.2 setting.

20) Remove supernatant and re-suspend in HBSS+(~200 $\mu$L/heart.

21) Transfer into small snap cap tubes for FACS, use 1 cc insulin syringe to remove any clumps of cells

22) Keep on ice from this point on (biologically inactive)

23) Remember to bring a 15 mL tube with 1 mL of media, to be used to collect cells.

24) Perform FACS analysis ASAP to minimize any changes that could occur over time.

Primary Cardiomyocyte Culture Medium

Medium should be made fresh before using but it can be stored for about a week in the fridge.

To 50 mL of DMEM-F12 add the following:

| | |
|---|---|
| d-glucose | 0.3 g |
| 1-glutamine | 500 $\mu$L of 200 mM stock |
| pen-strep | 500 $\mu$L of 5000 unit stock |
| insulin | 250 $\mu$L of 5mg/mL stock |
| apotransferrin | 100 $\mu$L of 50 mg/mL stock |
| progesterone | 1 $\mu$L of 1 mM stock |
| putrescine | 60 $\mu$L of 8 mg/mL stock |
| selenium | 15 $\mu$L of 100 $\mu$M stock |
| fungizone | 50 $\mu$L of 12.5 $\mu$g/mL stock |
| heparin | 10 $\mu$L of 10 mg/mL stock |

Filter medium with a 0.2 $\mu$m filter. Add 40 $\mu$L of 25 $\mu$g/mL bFGF stock after filtering. EGF is optional. If used include in medium before filtering at 10 $\mu$L of 100 $\mu$g/mL stock to 50 mL DMEM-F12.

RECIPES FOR CARDIOMYOCYTE ISOLATION

MEM-JM (500 mL)

400 mL dH2O
5.65 g MEM-JM
1.0 g Na bicarbonate
pH solution to 7.3 with NaOH, final volume of 500 mL with H2O, filter sterilize.

2X Collagenase (make fresh)

30 mg collagenase
15 mL MEM-JM
sterilize with filter (syringe)

DMEM -10% FBS

400 mL DMEM
45 mL FBS
4.5 mL pen/strep

DMEM-F12 + ITS (500 mL)

half package of DMEM-F 12 (~7.8 g)
2.5 mL pen/strep
500 $\mu$L ITS
0.6 g NaHCO$_3$

Primary Cardiomyocyte Isolation

Preparation:

Prepare media, add 30 mg collagenase to 15 mL JMEM, filter sterilize and leave in 37°C water bath. (~20 mice less than 6 days old)

Dissection of hearts:

1) Cut out hearts from mice, try to avoid the atria, place in 50 mL tube of cold DMEM-JM.
2) Wash hearts by pipetting up and down in JMEM media.
3) Transfer hearts to petri dish, keep moist with PBS if required.
4) Using two scalpel blades, mince up hearts.

Collagenase Digestion:

1) GENTLY wash the ventricular tissue in the petri dish with 20 mL JMEM using 25 mL wide mouth pipet. After a few cycles, pipet up tissue and media using pipet. Let tissue settle and "spit" out the tissue only into a clean 50 mL tube. Discard media, contains mostly red blood cells.
2) Using same 25 mL pipet, remove any remaining fluid from the tissue in the 50 mL tube and add appropriate volumes of JMEM and collagenase 2X. (see table, and consider the amount of tissue used).

| Volumes for 2 litters | | | | |
|---|---|---|---|---|
| DIGESTION # | Time (minutes) | JMEM (mL) | Collagenase 2X (mL) | Discard digestion ? |
| 0 | 15 | 5 | 3 | yes |
| 1 | 15 | 3 | 3 | no |
| 2 | 15 | 3 | 3 | no |
| 3 | 15 | 3 | 3 | no |
| 4 optional | 10 | 3 | 3 | no |

3) Incubate at 37 °C with a gentle 20 -30 rotations /minute for 15 minutes. Meanwhile prep 2 - 50 mL tubes with 10%FBS + DMEM.

4) Following the first incubation, SLOWLY triturate both tissue and media with a 25 mL pipet approximately 3 times. Wait 1 minute to let tissue settle and aspirate slowly the supernatant and discard (mostly RBC's and debris the first time). Replace with 10 mL of fresh JMEM. Repeat wash step once. Allow tissue to settle for 1 minute and discard supernatant. Measure out amount of tissue and add JMEM to adjust to the appropriate volume (see TABLE). Add the collagenase and incubate.

5) At then end of subsequent incubations, slowly suck up both tissue and media with 10 mL pipet and triturate 2 times. Wait 1 minute and slowly aspirate the supernatant and transfer to 50 mL tube containing 10 mL FBS to halt collagenase digestion. Replace host tissue with fresh 10 mL JMEM. Repeat wash step once. Allow tissue to settle for 1 minute and transfer supernatant to the same 50 mL tube with FBS. Measure out amount of tissue and add JMEM to adjust to the appropriate volume, then add collagenase.

6) To the 50 mL tube containing FBS and the supernatant from the two washing, add enough DMEM+10% FBS to fill up the tube. Centrifuge in the airplane at 50 g for 5 minutes. Vacuum aspirate the supernatant and resuspend the pellet in 10 mL DMEM-20% FBS. Store at room temperature in the hood.

7) Repeat steps 4 and 5 two (or three) more times.

8) Following the last incubation, slowly triturate twice with 10 mL pipet. Replace the host tissue with fresh 10 mL DMEM + 10% FBS, Repeat wash step once. Allow tissue to settle for one minute and transfer supernatant to the same 50 mL tube with FBS.

9) To the 50 mL tube containing FBS and supernatant from two washings add enough DMEM +10% FBS to fill up the tube and centrifuge at 500 g for 5 minutes.

Pre-plating Procedure:

1) Remove supernatant

2) Resuspend in 20 mL DMEM + 10% FBS.

3) Prepare for filtration by placing a nytex nylon filter over a sterile 50 mL tube. Disperse filtered suspension evenly over one sterile 150 mm petri dish and incubate at 37°C for 15 minutes. (THIS IS THE FIRST PLATING)

4) Following incubation, triturate slowly and transfer supernatant (~20 mL) to a second dish.

5) Wash old dish with fresh 10 mL DMEM + 10% FBS and transfer to a second dish (THIS IS SECOND PLATING) Incubate at 37°C for 15 minutes

6) Resuspend pellet with 10 mL or less of DMEM +10% FBS. Keep in mind that, lower amounts of resuspension volume means that more cells will be used during the cell counting procedure and that the concentration should be higher than the final concentration that will be used during the plating process.

7) Cells are plated on 2mm X 6mm dishes in ~5 mL of DMEM + 10% FBS. Incubate 20 hours on coated plates. The second plate of pre-plated cells were kept with DMEM + 10% FBS.

8) After 20 hour incubation change the media of the plates to DMEM F12 + ITS.

9) Following the first day of growth, rinse plates gently and transferred the growth media containing floating cells to 15 mL tubes. Spun cells down and re-plated on new DMEM + 10% FBS media on coated plates. The second pre-plate was grown in DMEM + 10% FBS for 3 days.

Cell Counting:

1) In a 1.5 mL eppendorftube, add 200 μL of cell suspension to 400 μL of 0.4% typan blue solution and 400 μL of DMEM + 10% FBS. Incubate for 2 minutes and count in hemocytometer. Count viable and non-viable cardiomyocytes separately. Viable myocytes should be fairly large and nucleated or multi-nucleated. Non-viable cells will be stained blue. Ignore RBC's and other small debris.

2) Following counting, and plating, incubate cells in pre-heated 37°C DMEM+ 10% FBS overnight or at least 16 hours. 24 hours is the maximum to incubate in the serum containing medium.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

The present invention has been described with regard to preferred embodiments. However, it will be obvious to persons skilled in the art that a number of variations and modifications can be made.

## Claims

1. An *ex vivo* method of promoting proliferation of a population of adult stem cells comprising contacting said stem cells with a polypeptide comprising a cardiotrophin-1 amino acid sequence, a derivative or active fragment thereof retaining substantially the same activity as the wild-type protein or a polynucleotide encoding said polypeptide.

2. Use of a polypeptide comprising a cardiotrophin-1 amino acid sequence, a derivative or active fragment thereof retaining substantially the same activity as the wild-type protein or a polynucleotide encoding said polypeptide for the preparation of a medicament for promoting proliferation of a population of adult stem cells.

3. Use of claim 2 for repairing or regenerating cardiac tissue.

4. The method according to claim 1 wherein said stem cell is a cardiac stem cell or a skeletal muscle stem cell.

5. The use according to claim 2, wherein said stem cell is a cardiac stem cell or a skeletal muscle stem cell.

6. The use according to claim 2, 3 or 5, further comprising use of one or more stem cell modulators that induce proliferation and/or differentiation of said stem cells for the preparation of the medicament.

7. The method of claim 1 or 4 for promoting adult stem cell proliferation and differentiation comprising contacting said stem cell with a first polypeptide comprising a cardiotrophin-1 amino acid sequence, a derivative or active fragment thereof retaining substantially the same activity as the wild-type protein or a polynucleotide encoding said first polypeptide, and one or more stem cell modulators, wherein said one or more stem cell modulators is a polypeptide or a polynucleotide encoding a polypeptide.

8. The use of claim 2, 3, 5 or 6, further comprising use of one or more stem cell modulators, wherein said one or more stem cell modulators is a polypeptide or a polynucleotide encoding a polypeptide for the preparation of a medicament, wherein the medicament is for promoting adult stem cell proliferation and differentiation.

9. The method according to claim 7, wherein said one or more stem cell modulators comprise at least one of a Wnt polypeptide or Pax7.

10. The use according to claim 8, wherein said one or more stem cell modulators comprise at least one of a Wnt polypeptide or Pax7.

11. The use of claim 2, wherein the medicament is suitable for subcutaneous injection, intramuscular injection, intraperitoneal injection, or intravenous injection.

12. The use of claim 11, wherein said intramuscular injection comprises intracardiac injection.

13. The use of claim 2, wherein said polynucleotide comprises a viral nucleotide sequence.

14. The use of claim 13, wherein said viral nucleotide sequence is an adenoviral or a retroviral nucleotide sequence.

**15.** The use of claim 13, wherein said nucleotide sequence comprises a secretion nucleotide sequence that promotes secretion of the peptide having cardiotrophin-1 activity from a cell in said subject.

**16.** The use of claim 15, wherein said secretion nucleotide sequence comprises a nerve growth signal sequence.

**17.** The use of any one of claims 11 to 16, wherein the medicament is suitable for administration comprising injecting two or more compositions, each of said compositions having cardiotrophin activity, and wherein said two or more injections are separated by a time interval of at least about 96 hours.

**18.** A polypeptide comprising a cardiotrophin-1 amino acid sequence, a derivative or active fragment thereof retaining the same activity as the wild-type protein, or a polynucleotide encoding said polypeptide for use in in vivo promoting proliferation of a population of adult stem cells.

**19.** A composition for use in in vivo promoting proliferation and/or differentiation of adult stem cells comprising,

a) a polypeptide comprising a cardiotrophin-1 amino acid sequence, a derivative or active fragment thereof retaining substantially the same activity as the wild-type protein, or a polynucleotide encoding said polypeptide, and;
b) at least one modulator of stem cell proliferation, differentiation or both selected from the group consisting of:

i) one or more Wnt polypeptides;
ii) one or more nucleotide sequences encoding one or more Wnt polypeptides;
iii) Pax 7 polypeptides;
iv) a nucleotide sequence encoding Pax 7 polypeptide;

or a combination thereof.

**20.** Products containing a first polypeptide comprising a cardiotrophin-1 amino acid sequence, a derivative or active fragment thereof retaining the same activity as the wild-type protein, or a polynucleotide encoding said first polypeptide and one or more stem cell modulators, wherein said one or more stem cell modulators is a polypeptide or a polynucleotide encoding a polypeptide as a combined preparation for simultaneous, separate or sequential use in in vivo promoting proliferation and/or differentiating adult stem cells.

**Patentansprüche**

**1.** Ex vivo-Verfahren zum Fördern der Proliferation einer Population von adulten Stammzellen, bei dem man die Stammzellen mit einem Polypeptid, umfassend eine Cardiotrophin-1-Aminosäuresequenz, ein Derivat oder aktives Fragment davon, das im Wesentlichen die gleiche Aktivität wie das Wildtyp-Protein beibehält, oder mit einem das Polypeptid codierenden Polynukleotid in Kontakt bringt.

**2.** Verwendung eines Polypeptids, umfassend eine Cardiotrophin-1-Aminosäuresequenz, ein Derivat oder aktives Fragment davon, das im Wesentlichen die gleiche Aktivität wie das Wildtyp-Protein beibehält, oder eines das Polypeptid codierenden Polynukleotids bei der Herstellung eines Arzneimittels für die Förderung der Proliferation einer Population von adulten Stammzellen.

**3.** Verwendung nach Anspruch 2 für die Reparatur oder Regeneration von Herzgewebe.

**4.** Verfahren nach Anspruch 1, wobei die Stammzelle eine Herzstammzelle oder eine Skelettmuskelstammzelle ist.

**5.** Verwendung nach Anspruch 2, wobei die Stammzelle eine Herzstammzelle oder eine Skelettmuskelstammzelle ist.

**6.** Verwendungen nach Anspruch 2, 3 oder 5, welche weiterhin die Verwendung von einem oder mehreren Stammzellenmodulatoren, der/die die Proliferation und/oder Differenzierung der Stammzellen induziert/induzieren, bei der Herstellung des Arzneimittels umfasst.

**7.** Verfahren nach Anspruch 1 oder 4 für die Förderung der Proliferation und Differenzierung von adulten Stammzellen, bei dem man die Stammzellen mit einem ersten Polypeptid, umfassend eine Cardiotrophin-1-Aminosäuresequenz, ein Derivat oder aktives Fragment davon, das im Wesentlichen die gleiche Aktivität wie das Wildtyp-Protein beibehält, oder mit einem das erste Polypeptid codierenden Polynukleotid und mit einem oder mehreren Stammzellenmodulator (en) in Kontakt bringt, wobei der eine oder die mehreren Stammzellenmodulator(en) ein Polypeptid oder ein ein Polypeptid codierendes Polynukleotid ist/sind.

8. Verwendung nach Anspruch 2, 3, 5 oder 6, bei der man weiterhin einen oder mehrere Stammzellenmodulator(en) verwendet, wobei der eine oder die mehreren Stammzellenmodulator(en) ein Polypeptid oder ein ein Polypeptid codierendes Polynukleotid ist/sind, für die Herstellung eines Arzneimittels, wobei das Arzneimittel für die Förderung der Proliferation und Differenzierung von adulten Stammzellen ist.

9. Verfahren nach Anspruch 7, wobei der eine oder die mehreren Stammzellenmodulator(en) wenigstens eines unter einem Wnt-Polypeptid oder Pax-7 umfasst/umfassen.

10. Verwendung nach Anspruch 8, wobei der eine oder die mehreren Stammzellenmodulator(en) wenigstens eines unter einem Wnt-Polypeptid oder Pax-7 umfasst/umfassen.

11. Verwendung nach Anspruch 2, wobei das Arzneimittel geeignet ist für die subkutane Injektion, die intramuskuläre Injektion, die intraperitoneale Injektion oder die intravenöse Injektion.

12. Verwendung nach Anspruch 11, wobei die intramuskuläre Injektion die intrakardiale Injektion umfasst.

13. Verwendung nach Anspruch 2, wobei das Polynukleotid eine virale Nukleotidsequenz umfasst.

14. Verwendung nach Anspruch 13, wobei die virale Nukleotidsequenz eine adenovirale oder eine retrovirale Nukleotidsequenz ist.

15. Verwendung nach Anspruch 13, wobei die Nukleotidsequenz eine Sekretionsnukleotidsequenz umfasst, die die Sekretion des Peptids mit Cardiotrophin-1-Aktivität aus einer Zelle in dem Patienten fördert.

16. Verwendung nach Anspruch 15, wobei die Sekretionsnukleotidsequenz eine Nervenwachstumssignalsequenz umfasst.

17. Verwendung nach einem der Ansprüche 11 bis 16, wobei das Arzneimittel geeignet ist für die Verabreichung, welche das Injizieren von zwei oder mehr Zusammensetzungen umfasst, wobei jede der Zusammensetzungen Cardiotrophin-Aktivität aufweist, und wobei die zwei oder mehr Injektionen durch einen Zeitintervall von wenigstens etwa 96 Stunden getrennt sind.

18. Polypeptid, umfassend eine Cardiotrophin-1-Aminosäuresequenz, ein Derivat oder aktives Fragment davon, das die gleiche Aktivität wie das Wildtyp-Protein beibehält, oder das Polypeptid codierendes Polynukleotid, für die Verwendung bei der Förderung der Proliferation einer Population von adulten Stammzellen *in vivo*.

19. Zusammensetzung für die Verwendung bei der Förderung der Proliferation und/oder Differenzierung von adulten Stammzellen *in vivo,* umfassend

   a) ein Polypeptid, umfassend eine Cardiotrophin-1-Aminosäuresequenz, ein Derivat oder aktives Fragment davon, das im Wesentlichen die gleiche Aktivität wie das Wildtyp-Protein beibehält, oder ein das Polypeptid codierendes Polynukleotid, und
   b) wenigstens einen Modulator der Stammzellenproliferation, -differenzierung oder von beidem, ausgewählt aus der Gruppe bestehend aus:

      i) eines oder mehrere Wnt-Polypeptide,
      ii) eines oder mehrere Nukleotidsequenzen, die eines oder mehrere Wnt-Polypeptide codieren,
      iii) Pax-7-Polypeptide,
      iv) eine Nukleotidsequenz, die ein Pax-7-Polypeptid codiert,

   oder eine Kombination davon.

20. Erzeugnisse, enthaltend ein erstes Polypeptid, umfassend eine Cardiotrophin-1-Aminosäuresequenz, ein Derivat oder aktives Fragment davon, das die gleiche Aktivität wie das Wildtyp-Protein beibehält, oder ein das erste Polypeptid codierendes Polynukleotid und einen oder mehrere Stammzellenmodulator(en), wobei der eine oder die mehreren Stammzellenmodulator(en) ein Polypeptid oder ein ein Polypeptid codierendes Polynukleotid ist/sind, als eine kombinierte Zubereitung für die gleichzeitige, getrennte oder aufeinanderfolgende Verwendung bei der Förderung der Proliferation und/oder Differenzierung von adulten Stammzellen *in vivo*.

**Revendications**

1. Procédé *ex vivo* d'activation de la prolifération d'une population de cellules souches adultes comprenant la mise en contact desdites cellules souches avec un polypeptide comprenant une séquence d'acide aminé de cardiotrophine-1, un dérivé ou un fragment actif de celle-ci ayant sensiblement la même activité que la protéine de type sauvage, ou un polynucléotide codant ledit polypeptide.

2. Utilisation d'un polypeptide comprenant une séquence d'acide aminé de cardiotrophine-1, un dérivé ou un fragment actif de celle-ci ayant sensiblement la même activité que la protéine de type sauvage, ou un polynucléotide codant ledit polypeptide pour la préparation d'un médicament destiné à activer la prolifération d'une population de cellules souches adultes.

3. Utilisation selon la revendication 2 pour réparer ou régénérer le tissu cardiaque.

4. Procédé selon la revendication 1, dans lequel ladite cellule souche est une cellule souche cardiaque ou une cellule souche du muscle squelettique.

5. Utilisation selon la revendication 2, dans laquelle ladite cellule souche est une cellule souche cardiaque ou une cellule souche du muscle squelettique.

6. Utilisation selon la revendication 2, 3 ou 5 comprenant en outre l'utilisation d'un ou plusieurs modulateurs de cellule souche induisant la prolifération et/ou la différenciation desdites cellules souches pour la préparation du médicament.

7. Procédé selon la revendication 1 ou 4, pour activer la prolifération et la différenciation de cellules souches adultes, comprenant la mise en contact de ladite cellule souche avec un premier polypeptide comprenant une séquence d'acide aminé de cardiotrophine-1, un dérivé ou un fragment actif de celle-ci ayant sensiblement la même activité que la protéine de type sauvage, ou un polynucléotide codant ledit premier polypeptide, et un ou plusieurs modulateurs de cellule souche, dans lequel lesdits un ou plusieurs modulateur(s) de cellule souche sont un polypeptide ou un polynucléotide codant un polypeptide.

8. Utilisation selon la revendication 2, 3, 5 ou 6, comprenant en outre l'utilisation d'un ou plusieurs modulateurs de cellule souche, dans laquelle ledit un ou plusieurs modulateurs de cellule souche est un polypeptide ou un polynucléotide codant un polypeptide pour la préparation d'un médicament, dans laquelle le médicament est destiné à activer la prolifération et la différenciation de cellules souches adultes.

9. Procédé selon la revendication 7, dans lequel lesdits un ou plusieurs modulateurs de cellule souche comprennent au moins un polypeptide Wnt ou Pax7.

10. Utilisation selon la revendication 8, dans laquelle lesdits un ou plusieurs modulateurs de cellule souche comprennent au moins un polypeptide Wnt ou Pax7.

11. Utilisation selon la revendication 2, dans laquelle le médicament est adapté pour une injection sous-cutanée, une injection intramusculaire, une injection intrapéritonéale, ou une injection intraveineuse.

12. Utilisation selon la revendication 11, dans laquelle ladite injection intramusculaire comprend une injection intracardiaque.

13. Utilisation selon la revendication 2, dans laquelle ledit polynucléotide comprend une séquence de nucléotide virale.

14. Utilisation selon la revendication 13, dans laquelle ladite séquence de nucléotide virale est une séquence de nucléotide adénovirale ou rétrovirale.

**15.** Utilisation selon la revendication 13, dans laquelle ladite séquence de nucléotide comprend une séquence de nucléotide de sécrétion, qui active la sécrétion du peptide ayant une activité de cardiotrophine-1 à partir d'une cellule dudit sujet.

**16.** Utilisation selon la revendication 15, dans laquelle ladite séquence de nucléotide de sécrétion comprend une séquence de signal de croissance nerveuse.

**17.** Utilisation selon l'une quelconque des revendications 11 à 16, dans laquelle le médicament est adapté pour une administration comprenant l'injection de deux compositions ou plus, chacune desdites compositions ayant une activité de cardiotrophine, et dans laquelle lesdites deux injections ou plus sont séparées par un laps de temps d'au moins environ 96 heures.

**18.** Polypeptide comprenant une séquence d'acide aminé de cardiotrophine-1, un dérivé ou fragment actif de celle-ci ayant la même activité que la protéine de type sauvage, ou un polynucléotide codant ledit polypeptide pour une utilisation dans l'activation *in vivo* de la prolifération d'une population de cellules souches adultes.

**19.** Composition pour une utilisation dans l'activation *in vivo* de la prolifération et/ou de la différenciation de cellules souches adultes comprenant :

a) un polypeptide comprenant une séquence d'acide aminé de cardiotrophine-1, un dérivé ou fragment actif de celle-ci ayant sensiblement la même activité que la protéine de type sauvage, ou un polynucléotide codant ledit polypeptide, et

b) au moins un modulateur de prolifération, de différenciation de cellules souches, ou les deux, choisies dans le groupe constitué de :

i) un ou plusieurs polypeptides Wnt ;
ii) une ou plusieurs séquences de nucléotides codant un ou plusieurs polypeptides Wnt;
iii) des polypeptides Pax 7 ;
iv) une séquence de nucléotide codant le polypeptide Pax 7 ;

ou une combinaison de ceux-ci.

**20.** Produits contenant un premier polypeptide comprenant une séquence d'acide aminé de cardiotrophine-1, un dérivé ou fragment actif de celle-ci ayant la même activité que la protéine de type sauvage, ou un polynucléotide codant ledit premier polypeptide et un ou plusieurs modulateurs de cellule souche, dans lequel ledit un ou plusieurs modulateurs de cellules souches est un polypeptide ou un polynucléotide codant un polypeptide, comme une préparation combinée pour une utilisation simultanée, séparée ou séquentielle dans l'activation *in vivo* de la prolifération et/ou la différenciation des cellules souches adultes.

Figure 1

Figure 2A-C

Figure 2D

Figure 2E

Figure 3A

33

## 24 hours ~ CTRL Heart

Figure 3B

## 24 hours ~ CT-1 Heart

Figure 3B Cont.

**48 hours ~ CTRL Heart**

Figure 3B Cont.

## 48 hours ~ CT-1 Heart

Figure 3B Cont.

**72 hours ~ CTRL Heart**

Figure 3B Cont.

## 72 hours ~ CT-1 Heart

Figure 3B Cont.

# Heart CTRL Injected 96 hours

Figure 3B Cont.

# Heart CT-1 Injected 96 hours

Figure 3B Cont.

## Average Heart Increase

Treated normalized against control, 5% error bar          n=4

# Figure 4

## Average Skeletal Muscle Increase

Treated normalized against control, 5% error bar          n=4

# Figure 5A

## Average Bone Marrow Increase

Treated normalized against control, 5% error bar          n=4

# Figure 5B

## Average Liver Increase

Treated normalized against control, 5% error bar          n=3

# Figure 5C

Age Course

Figure 6

Figure 7

**Adeno-cardiotrophin
to heart**

+

**cardiotoxin
to muscle**

→ **skeletal muscle
damage/repair**

**proof of principle**

Figure 8

Figure 9

49

control          ctx          ctx
                              +
                              CT-1

sections stained with a-actinin (z-bands)
(shows skeletal muscle sarcomere integrity)

Figure 10

## REFERENCES CITED IN THE DESCRIPTION

**Patent documents cited in the description**

- US 20030054973 A
- US 20030027330 A
- US 20030103951 A
- US 20020142457 A
- US 5534615 A
- US 5571675 A
- US 5571893 A
- US 5624806 A
- US 5679545 A
- US 20020146707 A
- US 5126249 A
- EP 495049 A
- US 4522752 A
- EP 45665 A, Szelke

- US 5723585 A, Pennica
- US 5627073 A
- US 6337184 B
- US 5679647 A
- US 5739118 A
- US 5837533 A
- WO 9206180 A
- WO 9222635 A
- WO 9220316 A
- WO 9314188 A
- WO 9320221 A
- WO 9325234 A
- WO 9406920 A

**Non-patent literature cited in the description**

- **Hierlihy, A.M. et al.** *FEBS Letters,* 2002, vol. 530, 239-243
- **Pennica, D. et al.** *Cytokine,* 1996, vol. 8, 183-189
- **Pennica, D. et al.** *Proc. Natl. Acad Sci. USA,* 1995, vol. 92, 1142-1146
- **Liao, Z. et al.** *Cardiovasc. Res.,* 2002, vol. 53, 902-919
- **Pennica, D. et al.** *Proc. Natl. Acad. Sci. USA,* vol. 92, 1142-1146
- **Sahm et al.** *J. Pharm. Pharmacol.,* 1996, vol. 48, 197
- **Audibert et al.** *Nature,* 1981, vol. 289, 593
- **Goodman et al.** *Perspectives in Peptide Chemistry,* 1981, 283-294
- **Spatola.** *Vega Data,* 1983, vol. 1 (3
- **Spatola.** Chemistry and Biochemistry of Amino Acids Peptides and Proteins. Marcel Dekker, 1983, 267
- **Morley, J. S.** *Trends Pharm. Sci.,* 1980, 463-468
- **Hudson et al.** *Int. J. Pept. Prot. Res.,* 1979, vol. 14, 177-185
- **Spatola et al.** *Life Sci.,* 1986, vol. 38, 1243-1249
- **Hann, J.** *Chem. Soc. Perkin Trans.,* 1982, I307-314
- **Almquist et al.** *J. Med Chem.,* 1980, vol. 23, 1392-1398
- **Jennings-White et al.** *Tetrahedron Lett.,* 1982, vol. 23, 2533

- **Holladay et al.** *Tetrahedron Lett.,* 1983, vol. 24, 4401-4404
- **Hruby.** *Life Sci.,* 1982, vol. 31, 189-199
- **James, G. L. et al.** *Science,* 1993, vol. 260, 1937-1942
- **Merrifield.** *J. Am. Chem. Soc.,* 1963, vol. 85, 2149
- **Merrifield.** *Science,* 1986, vol. 232, 341
- **Bittner et al.** *Methods in Enzymol.,* 1987, vol. 153, 516
- **Ausubel et al.** Current Protocols in Molecular Biology. John Wiley & Sons, 1994
- **Pouwels et al.** Cloning Vectors: A Laboratory Manual. 1985
- Current Protocols in Protein Science. J. Wiley & Sons
- **Maddox, D. E. et al.** *J. Exp. Med.,* 1983, vol. 158, 1211-1216
- **Hampton, R. et al.** Serological Methods: A Laboratory Manual. APS Press, 1990
- **Coligan, J. E. et al.** Current Protocols in Immunology. Wiley & Sons, 1997
- **Madlambayan, G.J. et al.** *J. Hematother. Stem Cell Res.,* 2001, vol. 10, 481-492
- **Hierlihy, A.M. et al.** *FEBS Lett.,* 2002, vol. 530, 239-243
- **Asakura, A. et al.** *J Cell Biol.,* 2002, vol. 159, 123-134
- **Gritti et al.** *J. Neurosci.,* 1999, vol. 19, 3287-3297

- **Hitoshi, S. et al.** *Genes & Dev.,* 2002, vol. 16, 846-858
- **Guo et al.** *Proc. Natl. Acad. Sci. USA,* 1999, vol. 96, 11507
- **Megeney et al.** *Genes Dev.,* 1996, vol. 10, 1173-1183
- **Asakura et al.** *J. Cell Biol.,* 2000, vol. 159, 123-134
- **Gennaro, A.** Remington: The Science and Practice of Pharmacy. Lippincott, William & Wilkins, 2000
- **Lesbordes et al.** *Hum. Molec. Gen.,* 2003, vol. 12, 1223-1229
- **Feigner ; Rhodes.** *Nature,* 1991, vol. 349, 351-352
- **Wu ; Wu.** *J Biol. Chem,* 1987, vol. 262, 4429-4432
- **Koller ; Smithies.** *Proc. Natl. Acad. Sci. USA,* 1989, vol. 86, 8932-8935
- **Zijlstra et al.** *Nature,* 1989, vol. 342, 435-438
- Viral Vectors: Basic Science and Gene Therapy. Eaton Publishing Co, 2000
- **Massie et al.** *Cytotechnology,* 1998, vol. 28, 53-64
- **Miller, A. D.** *Blood,* 1990, vol. 76, 271
- Current Protocols in Molecular Biology. J. Wiley & Sons, 1989
- **Miller.** *Human Gene Therapy,* 1990, vol. 1, 5-14
- **Roux et al.** *PNAS,* 1989, vol. 86, 9079-9083
- **Julan et al.** *J. Gen Virol,* 1992, vol. 73, 3251-3255
- **Goud et al.** *Virology,* 1983, vol. 163, 251-254
- **Neda et al.** *J Biol Chem,* 1991, vol. 266, 14143-14146
- **Berkner et al.** *BioTechniques,* 1988, vol. 6, 616
- **Rosenfeld et al.** *Science,* 1991, vol. 252, 431-434
- **Rosenfeld et al.** *Cell,* 1992, vol. 68, 143-155
- **Haj-Ahmand ; Graham.** *J. Virol.,* 1986, vol. 57, 267
- **Graham et al.** Methods in Molecular Biology. Humana, 1991, vol. 7, 109-127
- **Goodell, M. et al.** *J Exp Med,* 1996, vol. 183, 1797-806
- **Goodell et al.** *Nat. Med.,* 1997, vol. 3, 1337-1343
- **Jackson et al.** *Proc.Natl.Acad.Sci.USA,* 1999, vol. 96, 14482-14486